# EUROPEAN PATENT APPLICATION

(11) **EP 4 269 556 A1**
(43) Date of publication of application: **01.11.2023**
(21) Application number: 21914936.6
(22) Date of filing: 19.05.2021
(51) Int. Cl.: C12M 1/34, C12N 15/12, G01N 33/68, G01N 27/62, G01N 30/72

(54) **JUDGMENT SUPPORTING INFORMATION GENERATING METHOD, JUDGMENT SUPPORTING INFORMATION GENERATING SYSTEM, AND INFORMATION PROCESSING DEVICE**

(30) Priority: 28.12.2020 JP 2020219399
(71) Applicant: MCBI, Inc., Tokyo, 102-0072 (JP)
(72) Inventor: INOUE Makoto, Tsukuba-shi, Ibaraki 305-0035 (JP)
(74) Representative: Godemeyer Blum Lenze Patentanwälte Partnerschaft mbB - werkpatent
(86) International application number: PCT/JP2021/018970
(87) International publication number: WO 2022/145074

(57) **Abstract**

[Problems] The purpose of the present technology is to provide a technique for accurately determining cognitive impairment, cognitive decline, or risk of any of them.

[Solution] The present technology provides a determination supporting information generating method, comprising: an index value calculating step of calculating, based on amounts of two or more biomarkers contained in a biological sample derived from a human, (I) a first index value indicating the presence or absence of cognitive impairment or cognitive decline in the human and (II) a second index value indicating the degree of progression of cognitive impairment or cognitive decline in the human; and a supporting information generating step of generating information that supports determination of cognitive impairment, cognitive decline, or risk of any of them, based on the first index value and the second index value.

## Description

### Technical Field

The present technology relates to a method of generating information that supports determination of cognitive impairment, cognitive decline, or risk of any of them, an information generating system that generates the information, and an information processing device.

### Background Art

As a means for discriminating difference between a normal condition and a non-normal condition of a living organism using a sample that exhibits normal or non-normal condition of the living organism, the main conventional technology is a technique that has been generally used for in vitro diagnostic agents. Most of the in-vitro diagnostic agents are used for diagnostic tests by analyzing components in blood as biomarkers. In the prior art in this field, an amount of a single specific protein or a so-called oligopeptide with a molecular weight of 10,000 or less in blood is measured or, in a case of an enzyme protein, its activity is measured, and its diagnosis has been aided by an apparent difference between normal (healthy) samples and diseased samples. That is, an amount of a single specific protein or amounts of a plurality of specific proteins or specific oligopeptides or a degree of activity thereof in a certain number of biological samples derived from healthy and diseased patients are measured in advance, and a range of abnormal value and a range of normal value are decided. Next, a biological sample to be evaluated is measured by the same method, and examination evaluation is performed depending on whether the measurement result belongs to any of the decided range of abnormal value and the decided range of normal value.

Regarding biomarkers used for detecting cognitive impairment, for example, Patent Literature 1 below discloses (a) a biomarker for detecting a cognitive impairment disease consisting of an intact protein of Apolipoprotein A1 containing an amino acid sequence represented by SEQ ID NO: 1 or a partial peptide thereof, (b) a biomarker for detecting a cognitive impairment disease consisting of an intact protein of Transthyretin containing an amino acid sequence represented by SEQ ID NO: 2 or a partial peptide thereof, and (c) a biomarker for detecting a cognitive impairment disease consisting of an intact protein of Complement C3 containing an amino acid sequence represented by SEQ ID NO: 3 or a partial peptide thereof.

### Citation List

### Patent Literature

Patent Literature 1: International Publication No. 2014-207888

### Summary of Invention

### Technical Problem

An object of the present technology is to provide a technique for accurately determining cognitive impairment, cognitive decline, or risk of any of them. Cognitive impairment refers to a stage of mild cognitive impairment (hereinafter also referred to as MCI) or a stage of dementia.

### Solution to Problem

The present inventor has found that specific determination supporting information is suitable for accurately determining cognitive impairment, cognitive decline, or risk of any of them. The inventor has also found that certain biomarker combinations are suitable for accurately determining cognitive impairment, cognitive decline, or risk of any of them.

That is, the present technology provides
a determination supporting information generating method, comprising:
an index value calculating step of calculating, based on amounts of two or more biomarkers contained in a biological sample derived from a human,
   (I) a first index value indicating the presence or absence of cognitive impairment or cognitive decline in the human and
   (II) a second index value indicating the degree of progression of cognitive impairment or cognitive decline in the human; and
a supporting information generating step of generating information that supports determination of cognitive impairment, cognitive decline, or risk of any of them, based on the first index value and the second index value.

The amounts of the two or more biomarkers used in the index value calculating step may include amounts of two or more biomarkers among the following biomarkers (1) to (10):
(1) alpha-1-B-glycoprotein or its partial peptide;
(2) alpha-2-antiplasmin or its partial peptide;
(3) alpha-2-macroglobulin or its partial peptide;
(4) albumin or its partial peptide;
(5) apolipoprotein A1 or its partial peptide;
(6) apolipoprotein C1 or its partial peptide;
(7) complement component 3 or its partial peptide;
(8) complement component 4 gamma chain or its partial peptide;
(9) hemopexin or its partial peptide; and
(10) transthyretin or its partial peptide.

The first index value can be calculated based on amounts of two or more biomarkers among the biomarkers (1), (2), (3), (4), (6), (7), (8), and (9).

The second index value can be calculated based on amounts of two or more biomarkers among the biomarkers (1), (2), (3), (4), (6), (7), (8), and (9).

The first index value may be an index value indicating whether the human has mild cognitive impairment or dementia.

The second index value may be an index value indicating whether the cognitive impairment of the human is in a stage of mild cognitive impairment or in a stage of dementia.

The present technology also provides
a determination supporting information generating system comprising an information processing device that executes:
an index value calculating step of calculating, based on amounts of two or more biomarkers contained in a biological sample derived from a human,
   (I) a first index value indicating the presence or absence of cognitive impairment or cognitive decline in the human and
   (II) a second index value indicating the degree of progression of cognitive impairment or cognitive decline in the human; and
a supporting information generating step of generating information that supports determination of cognitive impairment, cognitive decline, or risk of any of them, based on the first index value and the second index value.

The determination supporting information generating system may further include a biomarker quantification system that quantifies amounts of the two or more biomarkers.

The biomarker quantification system may comprise a liquid chromatography mass spectrometer.

The system may be configured such that the biological sample is subjected to proteolytic degradation treatment and subjected to measurement by the biomarker quantification system.

The present technology also provides
an information processing device that executes:
an index value calculating step of calculating, based on amounts of two or more biomarkers contained in a biological sample derived from a human,
   (I) a first index value indicating the presence or absence of cognitive impairment or cognitive decline in the human and
   (II) a second index value indicating the degree of progression of cognitive impairment or cognitive decline in the human; and
a supporting information generating step of generating information that supports determination of cognitive impairment, cognitive decline, or risk of any of them, based on the first index value and the second index value.

### Advantageous Effects of Invention

The present technology contributes to accurately determining cognitive impairment, cognitive decline, or risk of any of them. The present technology also allows for determination of the degree of progression of cognitive impairment or cognitive decline in humans.

Note that an effect of the present technology is not necessarily limited to the effect described in this paragraph, and may be any of effects described in the present specification.

### Brief Description of Drawings

[Fig. 1] Fig. 1 is a flow diagram of an example of a judgment supporting information generating method of the present technology.
[Fig. 2] Fig. 2 is a flow diagram of an example of a data acquisition step.
[Fig. 3] Fig. 3 is a block diagram of an example of a biomarker quantification system.
[Fig. 4] Fig. 4 is a diagram showing a specific configuration example of an information processing device included in the biomarker quantification system.
[Fig. 5] Fig. 5 is a diagram for explaining a first index value.
[Fig. 6] Fig. 6 is a diagram for explaining a second index value.
[Fig. 7] Fig. 7 is a diagram for explaining judgment scores.
[Fig. 8] Fig. 8 is a diagram showing an example of plot data in which judgment scores are plotted.
[Fig. 9] Fig. 9 is a diagram showing an example of plot data containing a two-dimensional matrix.
[Fig. 10] Fig. 10 is a diagram showing a specific configuration example of an information processing device that executes an index value calculating step and a supporting information generating step.
[Fig. 11] Fig. 11 is a block diagram of an example of a judgment supporting information generating system.
[Fig. 12] Fig. 12 is a diagram showing evaluation results of the discriminant.
[Fig. 13] Fig. 13 is a diagram showing evaluation results of the discriminant.
[Fig. 14] Fig. 14 is a diagram showing evaluation results of the discriminant.
[Fig. 15] Fig. 15 is a diagram showing evaluation results of the discriminant.
[Fig. 16] Fig. 16 is a diagram showing an example of a table for explaining judgment results.
[Fig. 17] Fig. 17 is a diagram showing an example of a table for explaining judgment results.
[Fig. 18] Fig. 18 is a diagram showing an example of plot data in which judgment scores are plotted.
[Fig. 19] Fig. 19 is a diagram showing an example of plot data in which judgment scores are plotted.
[Fig. 20] Fig. 20 is a diagram showing an example of a table containing biomarker amount data.
[Fig. 21] Fig. 21 is a diagram showing an example of plot data regarding judgment results based on the amount of four biomarkers.

### Description of Embodiments

Hereinafter, embodiments for implementing the present technology will be described in detail. The embodiments described below show examples of typical embodiments of the present technology, and the present technology is not limited only to these embodiments.

### 1. Determination supporting information generating method

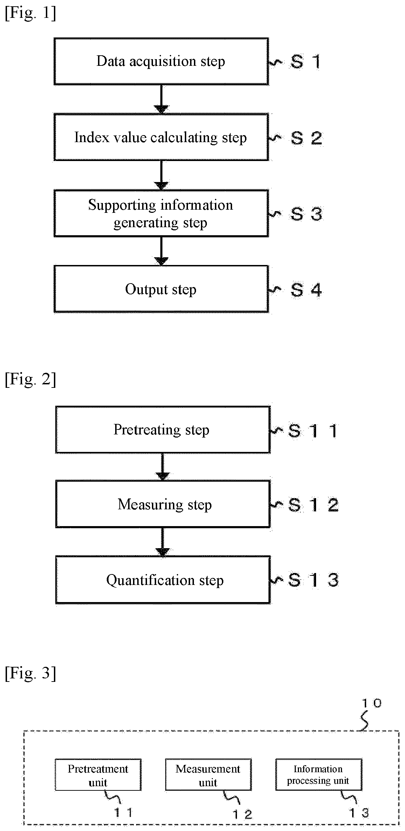

The present technology provides a determination supporting information generating method. The method may generate information that supports determination of cognitive impairment, cognitive decline, or risk of any of them. For example, as shown in Fig. 1, the method includes a data acquisition step S1 of acquiring data regarding amounts of two or more biomarkers contained in a biological sample derived from a human, an index value calculating step S2 of calculating, based on amounts of two or more biomarkers contained in the biological sample derived from the human, (I) a first index value indicating the presence or absence of cognitive impairment or cognitive decline in the human, and (II) a second index value indicating the degree of progression of cognitive impairment or cognitive decline in the human, and a supporting information generating step S3 of generating information that supports determination of cognitive impairment, cognitive decline, or risk of any of them, based on the first index value and the second index value. Furthermore, the method may comprise an output step S4 of outputting supporting information generated in the supporting information generating step S3.

In the present specification, cognitive impairment includes, for example, MCI and dementia. The dementia includes Alzheimer's disease (hereinafter also referred to as AD), dementia with Lewy bodies, and cerebrovascular dementia. In particular, the dementia is AD.

In the present specification, cognitive decline refers to a state of decrease in cognitive function, but not cognitive impairment.

In general, cognitively healthy humans, for example as they get older, first develop a state of cognitive decline and then suffer from cognitive impairment. Cognitive impairment often progresses gradually, and for example, humans first suffer from MCI and then dementia. The present technology allows for determining which stage in the progression a person is in.

These steps are described in more detail below.

### 1-1. Data acquisition step

In the data acquisition step S1, data to be used in the index value calculating step, which will be described later, is acquired. The data may include, for example, data relating to amounts of two or more biomarkers contained in a biological sample derived from a human.

The biological sample may be, for example, whole blood, plasma, or serum, preferably plasma or serum, particularly preferably plasma. That is, all of the biomarkers may be components present in a human-derived biological sample, and may be components present in particularly human whole blood, plasma, or serum, more preferably human plasma or serum, still more preferably human plasma.

### 1-1-1. Biomarkers

The amounts of the two or more biomarkers preferably includes amounts of two or more biomarkers among the following biomarkers (1) to (10):
(1) alpha-1-B-glycoprotein or a partial peptide thereof;
(2) alpha-2-antiplasmin or a partial peptide thereof;
(3) alpha-2-macroglobulin or a partial peptide thereof;
(4) albumin or a partial peptide thereof;
(5) apolipoprotein A1 or a partial peptide thereof;
(6) apolipoprotein C1 or a partial peptide thereof;
(7) complement component 3 or a partial peptide thereof;
(8) complement component 4 gamma chain or a partial peptide thereof;
(9) hemopexin or a partial peptide thereof; and
(10) transthyretin or a partial peptide thereof.

In the data acquisition step, an amount of a biomarker other than the above (1) to (10) may be acquired.

In the data acquisition step, the amounts of the biomarkers (1) to (10) may be quantified based on the amounts of the amino acid sequence portion specific to the protein described as each biomarker. The specific amino acid sequence portion may be an amino acid sequence portion that allows each biomarker to be distinguished from other components (more particularly biological sample components). The amino acid sequence portion may be appropriately selected by those skilled in the art. The amino acid sequence portion may be, for example, a protein fragment (also referred to as a quantitative fragment) to be measured by a mass spectrometer such as a liquid chromatography mass spectrometer.

Each of the partial peptides described in (1) to (10) above may be, for example, a peptide containing an amino acid sequence portion specific to each of the proteins described as biomarkers as described above.

Each of the partial peptides described in (1) to (10) above may be, for example, a peptide having 5 to 30, particularly 6 to 25, more particularly 7 to 20 amino acid residues.

Examples of the partial peptides described in each of the above (1) to (10) are quantitative fragments described in Table 1 below or partial peptides containing the amino acid sequences of the quantitative fragments, but are not limited thereto.

The alpha-1-B-glycoprotein described as the biomarker of (1) described above may be a protein containing an amino acid sequence having 95% or more, 96% or more, 97% or more, 98% or more, or 99% or more sequence identity with the amino acid sequence represented by SEQ ID NO: 1 below.

The alpha-2-antiplasmin described as the biomarker of (2) described above may be a protein containing an amino acid sequence having 95% or more, 96% or more, 97% or more, 98% or more, or 99% or more sequence identity with the amino acid sequence represented by SEQ ID NO: 2 below.

The alpha-2-macroglobulin described as the biomarker of (3) described above may be a protein containing an amino acid sequence having 95% or more, 96% or more, 97% or more, 98% or more, or 99% or more sequence identity with the amino acid sequence represented by SEQ ID NO: 3 below.

The albumin described as the biomarker of (4) described above may be a protein containing an amino acid sequence having 95% or more, 96% or more, 97% or more, 98% or more, or 99% or more sequence identity with the amino acid sequence represented by SEQ ID NO: 4 below.

The apolipoprotein A1 described as the biomarker of (5) described above may be a protein containing an amino acid sequence having 95% or more, 96% or more, 97% or more, 98% or more, or 99% or more sequence identity with the amino acid sequence represented by SEQ ID NO: 5 below.

The apolipoprotein C1 described as the biomarker of (6) described above may be a protein containing an amino acid sequence having 95% or more, 96% or more, 97% or more, 98% or more, or 99% or more sequence identity with the amino acid sequence represented by SEQ ID NO: 6 below.

The complement component 3 described as the biomarker of (7) described above may be a protein containing an amino acid sequence having 95% or more, 96% or more, 97% or more, 98% or more, or 99% or more sequence identity with the amino acid sequence represented by SEQ ID NO: 7 below.

The complement component 4 gamma chain described as the biomarker of (8) described above may be a protein containing an amino acid sequence having 95% or more, 96% or more, 97% or more, 98% or more, or 99% or more sequence identity with the amino acid sequence represented by SEQ ID NO: 8 below.

The hemopexin described as the biomarker of (9) described above may be a protein containing an amino acid sequence having 95% or more, 96% or more, 97% or more, 98% or more, or 99% or more sequence identity with the amino acid sequence represented by SEQ ID NO: 9 below.

The transthyretin described as the biomarker of (10) described above may be a protein containing an amino acid sequence having 95% or more, 96% or more, 97% or more, 98% or more, or 99% or more sequence identity with the amino acid sequence represented by SEQ ID NO: 10 below.

In the present specification, the term "sequence identity" of amino acid sequences means the following. Two amino acid sequences to be compared are aligned so that as many amino acid residues of the both amino acid sequences as possible are matched, the number of the matched amino acid residues is divided by the total number of amino acid residues, and the quotient is expressed as a percentage. The percentage is the sequence identity. The alignment and the percentage calculation may be performed using well-known algorithms such as BLAST and the like.

The amino acid sequences (single-letter code) of SEQ ID NOs: 1 to 10 above are as follows. Below, in parentheses after each SEQ ID NO, the name of the biomarker is described, as well as the abbreviations used in the present specification are described.

SEQ ID No. 1 (alpha-1-B-glycoprotein, A1BG): 
MSMLVVFLLLWGVTWGPVTEAAIFYETQPSLWAESESLLKPLANVTLTCQAHLETPDFQL
FKNGVAQEPVHLDSPAIKHQFLLTGDTQGRYRCRSGLSTGWTQLSKLLELTGPKSLPAPW
LSMAPVSWITPGLKTTAVCRGVLRGVTFLLRREGDHEFLEVPEAQEDVEATFPVHQPGNY
SCSYRTDGEGALSEPSATVTIEELAAPPPPVLMHHGESSQVLHPGNKVTLTCVAPLSGVD
FQLRRGEKELLVPRSSTSPDRIFFHLNAVALGDGGHYTCRYRLHDNQNGWSGDSAPVELI
LSDETLPAPEFSPEPESGRALRLRCLAPLEGARFALVREDRGGRRVHRFQSPAGTEALFE
LHNISVADSANYSCVYVDLKPPFGGSAPSERLELHVDGPPPRPQLRATWSGAVLAGRDAV
LRCEGPIPDVTFELLREGETKAVKTVRTPGAAANLELIFVGPQHAGNYRCRYRSWVPHTF
ESELSDPVELLVAES
SEQ ID No. 2 (alpha-2-antiplasmin, A2AP): 
MALLWGLLVLSWSCLQGPCSVFSPVSAMEPLGRQLTSGPNQEQVSPLTLLKLGNQEPGGQ
TALKSPPGVCSRDPTPEQTHRLARAMMAFTADLFSLVAQTSTCPNLILSPLSVALALSHL
ALGAQNHTLQRLQQVLHAGSGPCLPHLLSRLCQDLGPGAFRLAARMYLQKGFPIKEDFLE
QSEQLFGAKPVSLTGKQEDDLANINQWVKEATEGKIQEFLSGLPEDTVLLLLNAIHFQGF
WRNKFDPSLTQRDSFHLDEQFTVPVEMMQARTYPLRWFLLEQPEIQVAHFPFKNNMSFVV
LVPTHFEWNVSQVLANLSWDTLHPPLVWERPTKVRLPKLYLKHQMDLVATLSQLGLQELF
QAPDLRGISEQSLVVSGVQHQSTLELSEVGVEAAAATSIAMSRMSLSSFSVNRPFLFFIF
EDTTGLPLFVGSVRNPNPSAPRELKEQQDSPGNKDFLQSLKGFPRGDKLFGPDLKLVPPM
EEDYPQFGSPK
SEQ ID No. 3 (alpha-2-macroglobulin, A2M): 
MGKNKLLHPSLVLLLLVLLPTDASVSGKPQYMVLVPSLLHTETTEKGCVLLSYLNETVTV
SASLESVRGNRSLFTDLEAENDVLHCVAFAVPKSSSNEEVMFLTVQVKGPTQEFKKRTTV
MVKNEDSLVFVQTDKSIYKPGQTVKFRVVSMDENFHPLNELIPLVYIQDPKGNRIAQWQS
FQLEGGLKQFSFPLSSEPFQGSYKVVVQKKSGGRTEHPFTVEEFVLPKFEVQVTVPKIIT
ILEEEMNVSVCGLYTYGKPVPGHVTVSICRKYSDASDCHGEDSQAFCEKFSGQLNSHGCF
YQQVKTKVFQLKRKEYEMKLHTEAQIQEEGTVVELTGRQSSEITRTITKLSFVKVDSHFR
QGIPFFGQVRLVDGKGVPIPNKVIFIRGNEANYYSNATTDEHGLVQFSINTTNVMGTSLT
VRVNYKDRSPCYGYQWVSEEHEEAHHTAYLVFSPSKSFVHLEPMSHELPCGHTQTVQAHY
ILNGGTLLGLKKLSFYYLIMAKGGIVRTGTHGLLVKQEDMKGHFSISIPVKSDIAPVARL
LIYAVLPTGDVIGDSAKYDVENCLANKVDLSFSPSQSLPASHAHLRVTAAPQSVCALRAV
DQSVLLMKPDAELSASSVYNLLPEKDLTGFPGPLNDQDNEDCINRHNVYINGITYTPVSS
TNEKDMYSFLEDMGLKAFTNSKIRKPKMCPQLQQYEMHGPEGLRVGFYESDVMGRGHARL
VHVEEPHTETVRKYFPETWIWDLVVVNSAGVAEVGVTVPDTITEWKAGAFCLSEDAGLGI
SSTASLRAFQPFFVELTMPYSVIRGEAFTLKATVLNYLPKCIRVSVQLEASPAFLAVPVE
KEQAPHCICANGRQTVSWAVTPKSLGNVNFTVSAEALESQELCGTEVPSVPEHGRKDTVI
KPLLVEPEGLEKETTFNSLLCPSGGEVSEELSLKLPPNVVEESARASVSVLGDILGSAMQ
NTQNLLQMPYGCGEQNMVLFAPNIYVLDYLNETQQLTPEIKSKAIGYLNTGYQRQLNYKH
YDGSYSTFGERYGRNQGNTWLTAFVLKTFAQARAYIFIDEAHITQALIWLSQRQKDNGCF
RSSGSLLNNAIKGGVEDEVTLSAYITIALLEIPLTVTHPVVRNALFCLESAWKTAQEGDH
GSHVYTKALLAYAFALAGNQDKRKEVLKSLNEEAVKKDNSVHWERPQKPKAPVGHFYEPQ
APSAEVEMTSYVLLAYLTAQPAPTSEDLTSATNIVKWITKQQNAQGGFSSTQDTVVALHA
LSKYGAATFTRTGKAAQVTIQSSGTFSSKFQVDNNNRLLLQQVSLPELPGEYSMKVTGEG
CVYLQTSLKYNILPEKEEFPFALGVQTLPQTCDEPKAHTSFQISLSVSYTGSRSASNMAI
VDVKMVSGFIPLKPTVKMLERSNHVSRTEVSSNHVLIYLDKVSNQTLSLFFTVLQDVPVR
DLKPAIVKVYDYYETDEFAIAEYNAPCSKDLGNA
SEQ ID No. 4 (albumin, ALB):
SEQ ID No. 5 (apolipoprotein A1, APOA1):
SEQ ID No. 6 (apolipoprotein C1, APOC1): 
MRLFLSLPVLVVVLSIVLEGPAPAQGTPDVSSALDKLKEFGNTLEDKARELISRIKQSEL
SAKMREWFSETFQKVKEKLKIDS
SEQ ID No. 7 (complement component 3, C3):
SEQ ID No. 8 (complement component 4 gamma chain, C4G): 
EAPKVVEEQESRVHYTVCIWRNGKVGLSGMAIADVTLLSGFHALRADLEKLTSLSDRYVS
HFETEGPHVLLYFDSVPTSRECVGFEAVQEVPVGLVQPASATLYDYYNPERRCSVFYGAP
SKSRLLATLCSAEVCQCAEGKCPRQRRALERGLQDEDGYRMKFACYYPRVEYGFQVKVLR
EDSRAAFRLFETKITQVLHFTKDVKAAANQMRNFLVRASCRLRLEPGKEYLIMGLDGATY
DLEGHPQYLLDSNSWIEEMPSERLCRSTRQRAACAQLNDFLQEYGTQGCQV
SEQ ID No. 9 (hemopexin, HPX): 
MARVLGAPVALGLWSLCWSLAIATPLPPTSAHGNVAEGETKPDPDVTERCSDGWSFDATT
LDDNGTMLFFKGEFVWKSHKWDRELISERWKNFPSPVDAAFRQGHNSVFLIKGDKVWVYP
PEKKEKGYPKLLQDEFPGIPSPLDAAVECHRGECQAEGVLFFQGDREWFWDLATGTMKER
SWPAVGNCSSALRWLGRYYCFQGNQFLRFDPVRGEVPPRYPRDVRDYFMPCPGRGHGHRN
GTGHGNSTHHGPEYMRCSPHLVLSALTSDNHGATYAFSGTHYWRLDTSRDGWHSWPIAHQ
WPQGPSAVDAAFSWEEKLYLVQGTQVYVFLTKGGYTLVSGYPKRLEKEVGTPHGIILDSV
DAAFICPGSSRLHIMAGRRLWWLDLKSGAQATWTELPWPHEKVDGALCMEKSLGPNSCSA
NGPGLYLIHGPNLYCYSDVEKLNAAKALPQPQNVTSLLGCTH
SEQ ID No. 10 (transthyretin, TTR):

Examples of amino acid sequence portions (quantitative fragments) specific to the biomarkers (1) to (10) are shown in Table 1 below. The same table also lists the SEQ ID NO corresponding to the quantitative fragment for each biomarker.

### [Table 1]

**Table 1: Examples of quantitative fragments**

| Biomarker number | Biomarker name (protein name) | Abbreviation | Amino acid sequence of quantitative fragment | Sequence number |
|---|---|---|---|---|
| (1) | alpha-1-B-glycoprotein | A1BG | LETPDFQLFK | 11 |
| (2) | alpha-2-antiplasmin | A2AP | LGNQEPGGQTALK | 12 |
| (3) | alpha-2-macroglobulin | A2M | LLIYAVLPTGDVIGDSAK | 13 |
| (4) | albumin | ALB | LVNEVTEFAK | 14 |
| (5) | apolipoprotein A1 | APOA1 | ATEHLSTLSEK | 15 |
| (6) | apolipoprotein C1 | APOC 1 | TPDVSSALDK | 16 |
| (7) | complement component 3 | C3 | TGLQEVEVK | 17 |
| (8) | complement component 4 gamma chain | C4G | ITQVLIVTK | 18 |
| (9) | hemopexin | HPX | NFPSPVDAAFR | 19 |
| (10) | transthyretin | TTR | AADDTWEPFASGK | 20 |

The amino acid sequence portion used for quantification of each biomarker is not limited to the amino acid sequence portion shown in Table 1, and it may be appropriately selected by those skilled in the art depending on the method of proteolytic degradation treatment and the amino acid sequence of each biomarker, and the like.

As used herein, "amount" of a biomarker may be an absolute amount or a relative amount of the biomarker in a biological sample. The relative amount is, for example, concentration. For example, the concentration of a biomarker may be a mass of the biomarker relative to an amount (volume or mass) of the biological sample, and for example, molarity.

### 1-1-2. Acquisition of biomarker amount data by quantification

In the data acquisition step, an amount of each biomarker contained in a biological sample may be quantified. In order to perform the quantification, the data acquisition step includes, for example, a pretreating step S11 in which a pretreatment of a biological sample is performed, a measuring step S12 in which amounts of quantitative fragments contained in the pretreated biological sample are measured, and a biomarker quantification step S13 in which an amount of each biomarker is quantified based on the amounts of the quantitative fragments measured in the measuring step, as shown in Fig. 2. These steps are described below.

### 1-1-2-1. Pretreating step

In the pretreating step S11, for example, the biological sample may be subjected to a proteolytic degradation treatment. The proteolytic degradation treatment degrades the biomarkers to be quantified to generate protein fragments (quantitative fragments).

The proteolytic degradation treatment may be, for example, a proteolytic degrading agent-based degradation treatment or a degradation treatment by heat and pH.

The proteolytic degrading agent used in the proteolytic degrading agent-based degradation treatment may be, for example, an enzyme or a chemical degrading agent. The enzyme may be a protease and preferably includes an endopeptidase. The endopeptidase may be one or a combination of two or more selected from, for example, trypsin, Endoproteinase Lys-C, Endoproteinase Glu-C, and Endoproteinase Asp-N. Particularly preferably, the enzyme is an enzyme that hydrolyzes peptide bonds on the carboxy group side of basic amino acids (lysine and arginine), it may be more specifically trypsin. The chemical degrading agent may be, for example, cyanogen bromide. In the degradation treatment, the biological sample is contacted with these degrading agents to produce quantitative fragments.

In the degradation treatment by heat and pH, the biological sample may be heated to, for example, 60 °C to 140 °C, particularly 70 °C to 130 °C, more particularly 80 °C to 120 °C. The heating time may be, for example, 0.5 hours to 10 hours, especially 1 hour to 4 hours, more especially 1 hour to 3 hours.

In the degradation treatment by heat and pH, the biological sample is subjected to an environment of, for example, pH 5 or less, particularly pH 1 to 4, and more particularly pH 1 to 3. The biological sample be left in the environment for the heating time mentioned above. For pH adjustment, for example, an acid or alkali may be added to the biological sample.

In the degradation treatment, the biological sample is subjected to such heat and pH-based degradation treatment to generate quantitative fragments.

The proteolytic degradation treatment may be terminated by techniques known in the art.

For example, when enzymatic degradation treatment is performed, the proteolytic degradation treatment may be stopped by enzyme deactivation treatment. The enzyme deactivation treatment may be, for example, pH reduction treatment by adding formic acid or TFA to the biological sample, or heat treatment of the biological sample.

When thermal degradation treatment is performed, the degradation treatment may be stopped by stopping the heating treatment of the biological sample and changing the temperature of the biological sample to a temperature at which degradation does not proceed. A cooling treatment, for example, may be performed for this modification.

When the degradation treatment by pH is performed, the degradation treatment may be stopped by changing the pH of the biological sample to a pH at which the degradation does not progress. For changing the pH, for example, an alkali or acid addition treatment may be performed.

### 1-1-2-2. Measuring step

In the measuring step S 12, amounts of quantitative fragments contained in the pretreated biological sample is measured. The measurement may be performed using a liquid chromatography mass spectrometer, more specifically LC-MS or LC-MS/MS. Among treatments performed by the liquid chromatography mass spectrometer, the liquid chromatography treatment may be reversed-phase chromatography. Among the treatments by the liquid chromatography mass spectrometer, the mass spectrometry treatment may be performed by MS or MS/MS, preferably by MS/MS. For example, in MS/MS, two or more stages of mass separation using collision-induced dissociation (CID) are performed on a specific group of ions. More specifically, the mass spectrometry treatment may be performed by MRM (Multiple Reaction Monitoring). That is, the liquid chromatography mass spectrometer may be configured to perform mass spectrometry treatment by MRM.

### 1-1-2-3. Quantification step

In the quantification step S13, the amount of each biomarker is quantified based on the amount of the quantitative fragment measured in the measuring step. For example, the amount of the biomarker is quantified based on the amount of the quantitative fragment, the molecular weight of the quantitative fragment, and the molecular weight of the biomarker containing the quantitative fragment.

For example, in the quantification step S13, amounts of the biomarkers (1) to (10) (especially proteins described as biomarkers) may be quantified based on the amounts of the quantitative fragments described in Table 1 above.

### 1-1-2-4. System that performs biomarker amount data acquisition by quantification

In the present technology, a biomarker quantification system may quantify amounts of the two or more biomarkers. For example, the biomarker quantification system may be configured to perform the steps set forth in A. to C. above, or may be configured to perform one or two of these steps. An example of the biomarker quantification system is shown in Fig. 3. Fig. 3 is a block diagram of the system.

As shown in Fig. 3, the biomarker quantification system 10 may include a pretreatment unit 11, a measurement unit 12 and an information processing unit 13.

The pretreatment unit 11 may be configured to perform the pretreating step S11. The pretreatment unit may include, for example, a proteolytic degradation treatment unit that performs the proteolytic degradation treatment on the biological sample. The proteolytic degradation treatment unit may include, for example, a receiving unit that receives the biological sample or receives a container that contains the biological sample, a degrading agent addition device that adds a proteolytic degrading agent to the biological sample, and a stop treating device that performs a treatment to stop degradation with the degrading agent. Moreover, the pretreatment unit may include a temperature control device that controls the temperature of the biological sample in the receiving unit.

The degrading agent addition device may include, for example, a pipette device that adds a proteolytic degrading agent to the biological sample, and a drive unit that drives addition of the degrading agent by the pipette device.

The stop treating unit may include, for example, a pipette device that adds a drug for stopping degradation by the proteolytic degrading agent to the biological sample, and a drive unit that drives the addition by the pipette device.

A device known in the art may be employed as the temperature control device.

The measurement unit 12 may be configured to perform the measuring step S12. The measurement unit may include, for example, a liquid chromatography mass spectrometer. The liquid chromatography mass spectrometer may measure amounts of quantitative fragments contained in the biological sample pretreated by the pretreatment unit. The liquid chromatography mass spectrometer may be the device as described in the "B. Measuring step" above.

The information processing unit 13 may be configured to perform the quantification step S13. The information processing unit calculates an amount of each biomarker based on the amount of the quantitative fragment measured in the measuring step S12. The information processing unit may be, for example, an information processing device attached to the liquid chromatography mass spectrometer, or may be an information processing device not attached to the liquid chromatography mass spectrometer. The information processing device may receive amount data of the quantitative fragments measured by the liquid chromatography mass spectrometer, and quantify amounts of the biomarkers (1) to (10) (especially proteins described as biomarkers) based on the amount data. For the quantification, reference may be made to, for example, the molecular weight of the quantitative fragment and the molecular weight of the biomarker containing the quantitative fragment.

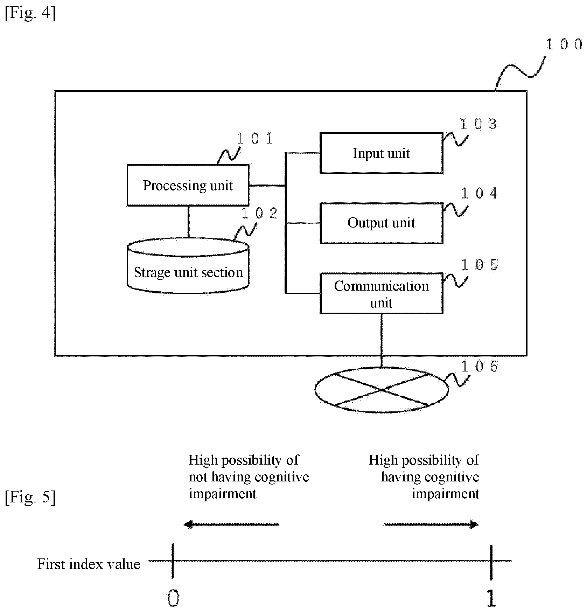

A concrete configuration example of the information processing device will be described with reference to Fig. 4. The information processing device 100 shown in Fig. 4 includes a processing unit 101, a storage unit 102, an input unit 103, an output unit 104, and a communication unit 105. For example, a general-purpose computer or server may be employed as the information processing device 100.

Processing unit 101 may, for example, be configured to perform the quantification step S13, and more specifically comprises a circuit configured to perform the quantification step S13. Processing unit 101 may include, for example, a CPU (Central Processing Unit) and a RAM. The CPU and RAM (Random Access Memory) may be interconnected, for example, via a bus. An input/output interface may be further connected to the bus. Input unit 103, output unit 104, and communication unit 105 may be connected to the bus via the input/output interface.

Processing unit 101 may further be configured to acquire data from the memory section 102 or record data in storage unit 102. Storage unit 102 stores various data. Storage unit 102 may be configured to store, for example, the measurement result data obtained in the measuring step 12 and the biomarker amount data quantified based on the measurement result data. In addition, storage unit 102 may store an operating system (for example, WINDOWS (registered trademark), UNIX (registered trademark), LINUX (registered trademark), or the like), a program for causing the information processing device to execute the quantification step, and other various programs. Note that these programs may be recorded in recording media, not limited to storage unit 102.

Processing unit 101 may be configured to be able to control the measurement unit 12 that executes the measuring step 12, and for example, processing unit 101 may control the liquid chromatography mass spectrometer included in the measurement unit 12. In addition, processing unit 101 may perform a process of calculating amounts of quantitative fragments based on data regarding ions detected by the liquid chromatography mass spectrometer.

Further, processing unit 101 may be configured to be able to control the pretreatment unit 11 that executes the pretreating step 11, and for example, processing unit 101 may control one or more of the degrading agent addition device, the stop treating device, and the temperature control device which are included in the pretreatment unit 11.

Input unit 103 may include an interface configured to receive input of various data. Input unit 103 may include, for example, a mouse, a keyboard, a touch panel, and the like as devices that receive such operations.

Output unit 104 may include an interface configured to output various data. For example, output unit 104 may output biomarker amount data obtained by quantification in the quantification step S13. Output unit 104 may include, for example, a display device and/or a printing device as a device that performs the output.

Communication unit 105 may be configured to connect the information processing device 100 to a network by wire or wirelessly. Communication unit 105 allows the information processing device 100 to acquire various data via the network. The acquired data may be stored in storage unit 102, for example. The configuration of communication unit 105 may be appropriately selected by those skilled in the art.

The information processing device 100 may transmit the acquired biomarker amount data to the information processing device that executes the index value calculating step S2, which will be described later, via communication unit 105, for example.

The information processing device 100 may include, for example, a drive (not shown), and the like. The drive may read data (for example, the various data mentioned above) or programs recorded on recording medium and output them to the RAM. The recording medium is, for example, a microSD memory card, an SD memory card, or a flash memory, but is not limited to these.

### 1-1-3. Acquisition of acquired biomarker amount data

In the data acquisition step, already acquired biomarker amount data may be acquired. In this embodiment, quantification as described in the above 1-1-2. need not be performed. In this embodiment, for example, an information processing device that executes an index value calculating step described later may receive biomarker amount data transmitted from another information processing device or biomarker amount data transmitted from a biomarker quantification system described later. Alternatively, an information processing device that executes an index value calculating step, which will be described later, may acquire biomarker amount data from a recording medium storing the biomarker amount data. These receiving processes or acquisition processes may be performed via a network.

### 1-2. Index value calculating step

In the index value calculating step S2, based on amounts of two or more biomarkers contained in a human-derived biological sample, (I) a first index value indicating the presence or absence of cognitive impairment or cognitive decline in the human, and (II) a second index value indicating the degree of progression of cognitive impairment or cognitive decline in the human is calculated. By using these two index values, highly accurate judgment can be performed in a supporting information generating step S3, which will be described later.

The amounts of the two or more biomarkers may be the data acquired in 1-1. Data acquisition step described above. For example, the information processing device may execute the index value calculating step of calculating the first index value and the second index value using the data.

### 1-2-1. First index value

The first index value may be an index value indicating whether the human has cognitive impairment or cognitive decline. The first index value may be calculated based on amounts of the two or more biomarkers, preferably may be calculated based on amounts of two or more, three or more, or four or more biomarkers among the biomarkers (1) to (10). The first index value can contribute to providing better accuracy in the supporting information generating step S3.

Further, the biomarkers used for calculating the first index value may be, for example, 9 or less, 8 or less, or 7 or less biomarkers among the biomarkers (1) to (10).

Preferably, the first index value may be calculated based on amounts of two or more, three or more, or four or more biomarkers among the biomarkers (1), (2), (3), (4), (6), (7), (8) and (9). Particularly preferably, the first index value may be calculated using amounts of each of four, five, six, seven, or eight biomarkers among the biomarkers (1), (2), (3), (4), (6), (7), (8), and (9).

Particularly preferably, at least amounts of each of the biomarkers (1), (4), (8) and (9) are used, for calculating the first index value.

For example, all of the biomarkers (1), (4), (8), and (9) and one, two, three, or four of the biomarkers (2), (3), (6), and (7) are used, for calculating the first index value.

Such biomarker combinations are useful as indicators of the presence or absence of cognitive impairment or cognitive decline, and are particularly useful as indicators of the presence or absence of cognitive impairment.

Particularly preferably, the first index value is calculated using amounts of each of the biomarkers (1), (4), (8), and (9), and amounts of each of the biomarkers (2) and (3), the biomarkers (2) and (6), the biomarkers (2) and (7), the biomarkers (3) and (6), the biomarkers (3) and (7), or the biomarkers (6) and (7). The first index value calculated using the amounts of these biomarkers is particularly suitable for judging cognitive impairment, cognitive decline, or risk of any of them.

In the present invention, the same biomarker combination or different biomarker combinations may be used for the calculation of the first index value for males and the first index value for females.

Also, in calculating the first index value, in addition to the combination of biomarkers, an amount(s) of the biomarkers (5) and/or (10) may be used, or may not be used.

The first index value may be calculated using, for example, one or more predetermined discriminants, particularly two or more predetermined discriminants. The discriminant(s) may be, for example, a discriminant(s) created by multivariate analysis, and in particular, may be a discriminant(s) obtained by performing multivariate analysis with the amount of each biomarker constituting the combination of biomarkers for calculating the first index value as an explanatory variable and with the presence or absence of cognitive impairment or cognitive decline as an objective variable.

In one embodiment of the present invention, the predetermined discriminants may be, for example, a discriminant prepared for each sex. That is, a discriminant for calculating the first index value for males and a discriminant for calculating the first index value for females may be prepared.

A good judgment result can be obtained with a discriminant prepared without distinguishing between sexes, or with a discriminant prepared for each sex.

The number of discriminants used to calculate the first index value may correspond to, for example, the number of stages of the degree of progression of cognitive impairment indicated by the second index value described later. For example, if the stage of cognitive impairment indicated by the second index value described later is two stages, MCI and AD, the number of discriminants used to calculate the first index value is also two. In this case, one of the two discriminants may be a discriminant for discrimination between the healthy stage and the MCI stage, and the other may be a discriminant for discrimination between the healthy stage and the AD stage.

The number of discriminants may correspond to the number of stages of the degree of progression of cognitive impairment indicated by the second index value, as described above, in both the case of preparing discriminants without distinguishing between sexes and the case of preparing discriminants for each sex.

When sex is not discriminated and when there are two stages of cognitive impairment indicated by the second index value described later, MCI and AD, the number of discriminants is two as described above.

When discriminants are prepared for each sex and the stage of cognitive impairment indicated by the second index value described later is two stages, MCI and AD, two discriminants are prepared to calculate the first index value for males, and two discriminants are also prepared to calculate the first index value for females. In this way, discriminants are prepared for each sex, and the number of the discriminants corresponds to the number of stages of the degree of progression of cognitive impairment indicated by the second index value.

In order to calculate the first index value, using the value obtained by substituting the biomarker amount into each of the one or more predetermined discriminants, a value may be calculated that indicates a likelihood relating to whether or not the person is at each stage of the degree of progression (e.g., MCI stage, AD stage, etc.). For example, to calculate the first index value, a probability relating to whether or not the person is in the MCI stage and a probability relating to whether or not the person is in the AD stage may be calculated. Using these probabilities, the first index value may be calculated.

For example, the first index value may be the sum of values indicating likelihood relating to whether or not the person is at each stage of the degree of progression (e.g., MCI stage, AD stage, etc.).

When using discriminants prepared without distinguishing between sexes, the calculation of the first index value may be performed as described above.

When using the discriminant prepared for each sex, if the subject is male, the biomarker amount is substituted into the male discriminant. In contrast, when the subject is female, the biomarker amount is substituted into the female discriminant. As a result, a value indicating likelihood is calculated as described above.

A population of humans known to have or not have cognitive impairment or cognitive decline may be used to create the discriminant. The number of humans constituting the population may be, for example, 50 or more, 60 or more, or 70 or more. Although the upper limit of the number of humans constituting the population is not particularly limited, it may be, for example, 500 or less, 400 or less, 300 or less, or 200 or less. By performing multivariate analysis using the presence or absence of cognitive impairment or cognitive decline in each human constituting the population and amounts of biomarkers contained in the biological samples obtained from each human, a discriminant(s) for calculating first index value is(are) obtained, and, in particular, the coefficient of each term (and the value of the constant term) of the discriminant(s) is obtained.

When preparing a discriminant that does not discriminate between sexes, the population for creating the discriminant may be as described above.

When preparing a discriminant for each sex, a population consisting of the above-mentioned number of males is prepared in order to create a discriminant for males. In contrast, in order to create a discriminant for females, a population consisting of the above-mentioned number of females is prepared. By performing the multivariate analysis described above for each population, the discriminant (particularly, the coefficient of each term (and the value of the constant term) of the discriminant) is obtained.

The multivariate analysis may preferably be logistic regression analysis (especially multinomial logistic regression analysis). Also, the multivariate analysis may be another linear regression analysis. The multivariate analysis may also be multi-class classification, and for example, machine learning models such as neural networks or support vector machines may be used. The first index value may be obtained using the machine learning models.

The first index value may be an index value indicating whether the human has cognitive impairment, and particularly may be an index value indicating whether the human has MCI or dementia (particularly AD).

The first index value may take a value within a predetermined value range. The predetermined value range may be a value range indicating that the closer the first index value is to the value of one end point of the value range, the more likely the human does not have cognitive impairment or cognitive decline, and the closer the first index value is to the value of the other end point of the value range, the more likely the human has cognitive impairment or cognitive decline.

The predetermined value range may be appropriately set by those skilled in the art. One endpoint of the predetermined value range may be -100, -50, -10, -5, -1, 0, 1, 5, 10, 50, or 100, for example. The other endpoint of the prescribed value range may be 100, 50, 10, 5, 1, 0, -1, -5, -10, -50, or -100. The predetermined value range may be a range defined by these endpoints, such as 0 to 1, 0 to 50, 0 to 100, -1 to 1, or -100 to 100, but the values of the endpoints of the value range may be values other than these.

For example, as shown in Fig. 5, when the predetermined value range is 0 to 1, it may be meant that the closer the first index value is to 0 (one end point), the more likely the person does not have cognitive impairment (or cognitive decline), and that the closer the first index value is to 1 (the other endpoint), the more likely the person has cognitive impairment (or cognitive decline).

Conversely, when the predetermined value range is 0 to 1, it may be meant that the closer the first index value is to 1 (one endpoint), the more likely the person does not have cognitive impairment (or cognitive decline), and that the closer the first index value is to 0 (the other endpoint), the more likely the person has cognitive impairment (or cognitive decline).

For example, the value range of the first index value may be the same as the value range of the second index value described below. As described above, since the first index value and the second index value have the same value range (numerical range), it becomes easier to execute the processing in the supporting information generating step, which will be described later. A person skilled in the art can appropriately set the discriminant in order to set these two value ranges to be the same.

### 1-2-2. Second index value

The second index value is an index value indicating the degree of progression of cognitive impairment or cognitive decline in the human. The second index value may be calculated based on amounts of the two or more biomarkers, and preferably may be calculated based on amounts of two or more, three or more, or four or more biomarkers among the biomarkers (1) to (10). The biomarkers used for calculating the second index value particularly comprise at least one biomarker different from the biomarkers used for calculating the first index value. The second index value can contribute to providing better accuracy in the supporting information generating step S3.

Further, the biomarkers used for calculating the second index value may be, for example, 9 or less, 8 or less, or 7 or less biomarkers among the biomarkers (1) to (10).

Furthermore, the combination of biomarkers used to calculate the second index value may be different from the combination of biomarkers used to calculate the first index value.

Preferably, the second index value may be calculated based on amounts of two or more, three or more, or four or more biomarkers among the biomarkers (1), (2), (3), (4), (6), (7), (8) and (9). Particularly preferably, the second index value may be calculated using amounts of each of four, five, six, seven, or eight biomarkers among the biomarkers (1), (2), (3), (4), (6), (7), (8) and (9).

For example, all of the biomarkers of (1), (4), (8), and (9) and one, two, three, or four of the biomarkers of (2), (3), (6), and (7) are used to calculate the first index value.

Such a biomarker combination is useful as an index regarding the degree of progression of cognitive impairment or cognitive decline, and particularly useful as an index of the degree of progression of cognitive impairment.

Particularly preferably, the second index value is calculated using the amount of each of the biomarkers (1), (4), (8), and (9), and amounts of each of the biomarkers (2) and (3), the biomarkers (2) and (6), the biomarkers (2) and (7), the biomarkers (3) and (6), the biomarkers (3) and (7), or the biomarkers (6) and (7). The second index value calculated using the amount of these biomarkers is particularly suitable for judging cognitive impairment, cognitive decline, or risk of any of them.

The combination of biomarkers used to calculate the second index value may be the same as or different from the combination of biomarkers used to calculate the first index value. When these combinations are the same, the method for calculating the first index value and the method for calculating the second index value may be different.

Also, in the present invention, the same biomarker combination may be used or different biomarker combinations may be used, for the calculation of the first index value for males and the first index value for females.

Further, in calculating the second index value, in addition to the combination of biomarkers, an amount(s) of the biomarkers (5) and/or (10) may be used, or may not be used.

Like the first index value, the second index value may also be calculated using one or more predetermined discriminants, particularly two or more predetermined discriminants. The discriminant(s) may be, for example, a discriminant(s) created by multivariate analysis, and in particular, may be a discriminant(s) obtained by performing multivariate analysis with the amount of each biomarker as an explanatory variable and with the degree of progression of cognitive impairment or cognitive decline as an objective variable.

In one embodiment of the present invention, the predetermined discriminants used for calculating the second index value may be, for example, a discriminant prepared for each sex. That is, a discriminant for calculating the second index value for males and a discriminant for calculating the second index value for females may be prepared.

A good judgment result can be obtained with a discriminant prepared without distinguishing between sexes, or with a discriminant prepared for each sex.

The number of discriminants used to calculate the second index value may correspond to, for example, the number of stages of the degree of progression of cognitive impairment indicated by the second index value. For example, when there are two stages, MCI and AD, the number of discriminants used to calculate the second index value may also be two. In this case, one of the two discriminants may be a discriminant for discrimination between the healthy stage and the MCI stage, and the other may be a discriminant for discrimination between the healthy stage and the AD stage.

The one or more predetermined discriminants used to calculate the second index value may be the same as the one or more predetermined discriminants used to calculate the first index value, or may be different. If these discriminants are the same, for example, the usages of probabilities for calculating these index values may be different.

The number of discriminants may correspond to the number of stages of the degree of progression of cognitive impairment indicated by the second index value, as described above, in both the case of preparing discriminants without distinguishing between sexes and the case of preparing discriminants for each sex.

When sex is not discriminated and when the stage of cognitive impairment indicated by the second index value is two stages of MCI and AD, the number of discriminants is two as described above.

When discriminants are prepared for each sex and when the stage of cognitive impairment indicated by the second index value is two stages, MCI and AD, two discriminations used to calculate the first index value for males are prepared, and two discriminants used to calculate the first index value for females are also prepared. In this way, discriminants are prepared for each sex, and the number of the discriminants corresponds to the number of stages of the degree of progression of cognitive impairment indicated by the second index value.

In order to calculate the second index value, using the value obtained by substituting the biomarker amount into each of the one or more predetermined discriminants, a value may be calculated that indicates a likelihood relating to whether or not the person is at each stage of the degree of progression (e.g., MCI stage, AD stage, etc.). For example, to calculate the second index value, a probability relating to whether or not the person is in the MCI stage and a probability relating to whether or not the person is in the AD stage may be calculated.

For example, the second index value may be a value indicating a likelihood relating whether or not the person is in any one stage among values indicating a likelihood relating to whether the person is in each stage of the degree of progression. For example, the second index value may be a value indicating a likelihood relating to whether or not the person is in an AD stage.

When using discriminants prepared without distinguishing between sexes, the calculation of the second index value may be performed as described above.

When using the discriminant prepared for each sex, if the subject is male, the biomarker amount is substituted into the male discriminant. In contrast, when the subject is female, the biomarker amount is substituted into the female discriminant. As a result, a value indicating likelihood is calculated as described above.

A population of humans whose degree of progression of cognitive impairment or cognitive decline are known may be used to create the discriminant. The population may preferably be the same as the population used to create the discriminant used to calculate the first index value. The number of humans constituting the population may be, for example, 50 or more, 60 or more, or 70 or more. Although the upper limit of the number of humans constituting the population is not particularly limited, it may be, for example, 500 or less, 400 or less, 300 or less, or 200 or less. By performing multivariate analysis using the degree of progression of cognitive impairment or cognitive decline of each human constituting the population and the amount of biomarkers contained in the biological sample obtained from each human, a discriminant(s) for calculating the second index value is(are) obtained, and, in particular, the coefficient of each term (and the constant term) of the discriminant(s) is obtained.

When preparing a discriminant that does not discriminate between sexes, the population for creating the discriminant may be as described above.

When preparing a discriminant for each sex, a population consisting of the above-mentioned number of males is prepared in order to create a discriminant for males. In contrast, in order to create a discriminant for females, a population consisting of the above-mentioned number of females is prepared. By performing the multivariate analysis described above for each population, the discriminant (particularly, the coefficient of each term (and the value of the constant term) of the discriminant) is obtained.

The multivariate analysis may preferably be logistic regression analysis (especially multinomial logistic regression analysis). Further, the multivariate analysis may be another linear regression analysis. The multivariate analysis may also be multi-class classification, and for example, machine learning models such as neural networks or support vector machines may be used. The second index value may be acquired using the machine learning models.

The degree of progression of the cognitive impairment may be, for example, a degree that indicates which stage a human is in, MCI stage or dementia stage. The dementias include AD, dementia with Lewy bodies, and cerebrovascular dementia. The dementia is in particular AD.

In one embodiment of the present technology, the second index value may be an index value indicating which stage a human is in, MCI stage or AD stage. In this embodiment, the discriminant(s) for calculating the index value can be obtained by the discriminant creation method described above.

In another embodiment of the present technology, the second index value may be an index value indicating which stage a human is in, early MCI stage, late MCI stage, early AD stage, or late AD stage. Also in this embodiment, the discriminant(s) for calculating the index value can be obtained by the discriminant creation method described above. For example, a discriminant(s) can be prepared using a population of humans known to be in any of these four stages.

Each stage of cognitive impairment indicated by the second index value may be associated with an evaluation index according to the Clinical Dementia Rating (CDR), for example. Evaluation indices according to CDR are values based on clinical information such as neuropsychological tests and daily living activity indices. In the CDR, a numerical value of 0 corresponds to healthy, a numerical value of 0.5 corresponds to MCI, and a numerical value of 1 to 3 corresponds to AD. For a score of 1-3, higher numbers mean more severe AD.

The second index value may be any value within a predetermined value range. The predetermined value range may be a value range indicating that the closer the second index value is to the value of one end point of the value range, the higher the possibility that the cognitive impairment or cognitive decline of the human is not progressing, and that the closer the second index value is to the value of the other end point of the value range, the higher the possibility that the cognitive impairment or cognitive decline of the human is progressing.

In one embodiment of the present technology, the predetermined value range may be a value range indicating that the closer the second index value is to the value of one end point of the value range, the higher the possibility that the human is in the stage of MCI is, and that the closer the second index value is to the value of the other end point of the value range, the higher the possibility that the human is in the stage of dementia (especially AD) is.

In another embodiment of the present technology, the predetermined value range may be a value range indicating that the closer the second index value is to the value of one end point of the value range, the higher the possibility that the human is in the stage of earlier MCI is, and that the closer to the other end point, the higher the possibility that the human is in the stage of later MCI, or the stage of later dementia is.

In this embodiment, it may be a value range indicating that the closer the second index value is to the value of the other end point of the value range, the higher the possibility that the human is in the stage of later dementia is and that the closer to the one end point, the higher the possibility that the human is in the stage of earlier dementia or in the stage of earlier MCI is.

The predetermined value range may be appropriately set by those skilled in the art. One endpoint of the predetermined value range may be -100, -50, -10, -5, -1, 0, 1, 5, 10, 50, or 100, for example. The other endpoint of the prescribed value range may be 100, 50, 10, 5, 1, 0, -1, -5, -10, -50, or -100. The predetermined value range may be a range defined by these endpoints, such as 0 to 1, 0 to 50, 0 to 100, -1 to 1, or -100 to 100.

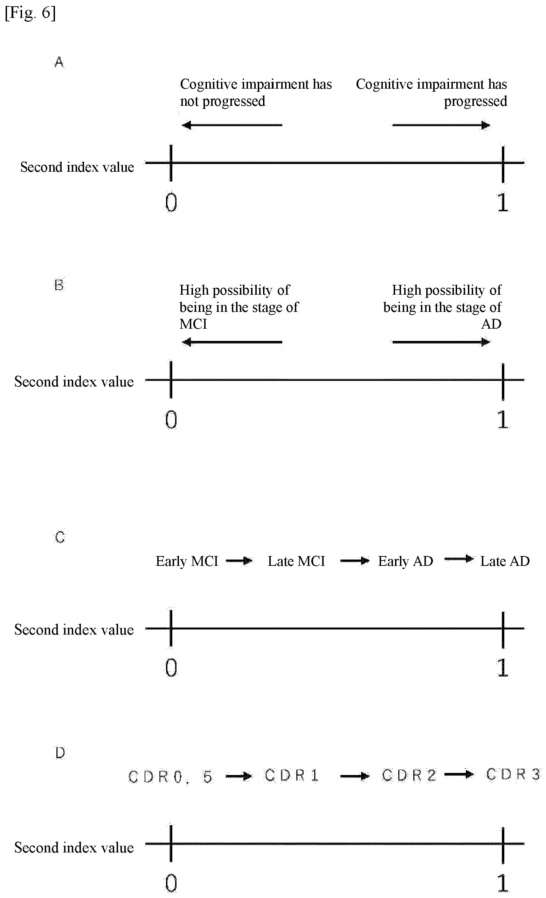

For example, as shown in Fig. 6A, when the predetermined value range is 0 to 1, it may be meant that the closer the second index value is to 0 (one end point), the higher the possibility that cognitive impairment or cognitive decline of the human is not progressing, and that the closer the second index value is to 1 (the other end point), the higher the possibility that cognitive impairment or cognitive decline of the human is progressing.

Conversely, when the predetermined value range is 0 to 1, it may be meant that the closer the second index value is to 1 (one end point), the higher the possibility that cognitive impairment or cognitive decline of the human is not progressing is, and that the closer the second index value is to 0 (the other end point), the higher the possibility that cognitive impairment or cognitive decline of the human is progressing is.

For example, as shown in Fig. 6B, when the predetermined value range is 0 to 1, it may be meant that the closer the second index value is to 0 (one end point), the higher the possibility that the human is in the stage of MCI is, and that the closer the second index value is to 1 (the other end point), the higher the possibility that the human is in the stage of dementia (especially AD) is.

Conversely, when the predetermined value range is 0 to 1, it may be meant that the closer the second index value is to 1 (one end point), the higher the possibility that the human is in the stage of MCI is, and that the closer the second index value is to 0 (the other end point), the higher the possibility that the human is in the stage of dementia (especially AD) is.

For example, as shown in Fig. 6C, when the predetermined value range is 0 to 1, the second index value may indicate whether the patient is likely to be in any stage of MCI (e.g., early stage or late stage), or in any stage of dementia (especially AD) (e.g., early stage or late stage). More specifically, cognitive impairment progresses from early MCI, late MCI, early AD, and late AD, as shown in Figure 6C. It may be meant that the closer the second index value is to 0 (one endpoint), the higher the possibility that the human is in the stage of earlier MCI is, and that the closer the second index value is to 1 (the other end point), the higher the possibility that the human is in the stage of later dementia (especially AD) is.

Conversely, when the predetermined value range is 0 to 1, it may be meant that the closer the second index value is to 1 (one endpoint), the higher the possibility that the human is in the stage of earlier MCI is, and that the closer the second index value is to 0 (the other end point), the higher the possibility that the human is in the stage of later dementia (particularly AD) is.

For example, as shown in Fig. 6D, when the prescribed value range is 0 to 1, the second index value may indicate which stage of the CDR evaluation score described above is likely to correspond to the score. As noted above, the CDR judges that cognitive impairment corresponds to a score of 0.5, corresponding to MCI, and anywhere from 1 to 3, corresponding to AD. That is, CDR progresses to 0.5, 1, 2, and 3 as cognitive impairment progresses. It may be meant that the closer the second index value is to 0 (one end point), the higher the possibility that the CDR of the human is closer to 0.5 (it may mean that the possibility of closer to 0 is high) is, and that the closer the second index value is to 1 (the other endpoint), the higher the possibility that the human CDR is closer to 3 is.

Conversely, when the prescribed value range is 0 to 1, it may be meant that the closer the second index value is to 1 (one endpoint), the higher the possibility that the human CDR is closer to 3 is, and that the closer the second index value is to 0 (the other end point), the higher the possibility that the human CDR is closer to 0.5 (it may mean that the possibility of closer to 0 is high) is.

### 1-3. Supporting information generating step

In the supporting information generating step S3, supporting information for determining cognitive impairment, cognitive decline, or risk of any of them is generated based on the first index value and the second index value. By using these two index values, supporting information that contributes to accurately determining cognitive impairment, cognitive decline, or risk of any of them is generated.

The supporting information includes, for example, one or more of determination results regarding cognitive impairment, determination results regarding cognitive decline, determination results regarding risk of cognitive impairment or cognitive decline, and data used for these determinations (for example, a score for determination and plot data, which are described later).

Further, the supporting information may further include data regarding an amount of each biomarker. The supporting information may further include determination results based on respective amount of each biomarker.

The supporting information will be described below.

### 1-3-1. determination results regarding cognitive impairment

The supporting information may include a determination result as to whether the human from whom the biological sample is derived has cognitive impairment and/or a determination result as to the degree of progression of cognitive impairment of the human.

In addition, the supporting information may include a determination result regarding whether symptoms of cognitive impairment are observed in the biological sample, and/or a determination result regarding symptoms indicating which stage of cognitive impairment is present in the biological sample among a plurality of stages of progression. By using the first index value and the second index value, highly accurate determination results can be obtained.

Conditions regarding cognitive function in humans, for example, transition from normal to MCI, and further from MCI to dementia (e.g., AD). Therefore, the supporting information may include a determination result as to which stage of a healthy stage, an MCI stage, and a dementia stage (particularly, an AD stage) the human is in. By using the first index value and the second index value, a highly accurate determination result can be obtained.

MCI can be classified into multiple stages, such as early MCI and late MCI, or classified in any of early MCI, intermediate MCI, and late MCI. Dementia is also classified into multiple stages, and for example, AD is classified into any of early AD and late AD, or classified into any of early AD, intermediate AD, and late AD. The supporting information may include a determination result as to which stage in the course of healthy, MCI, and dementia (e.g., AD) the human is in. The method of classifying the stages of MCI and dementia (e.g., AD) may be, for example, as described above, but is not limited to this.

### 1-3-2. Determination results regarding cognitive decline

The supporting information may include a determination result as to whether the human from whom the biological sample is derived has cognitive decline and/or a determination result as to the degree of progression of cognitive decline in the human.

In addition, the supporting information may include a determination result regarding whether symptoms of cognitive decline are observed in the biological sample, and/or a determination result regarding symptoms indicating which stage of cognitive decline is present in the biological sample among a plurality of stages of progression. By using the first index value and the second index value, highly accurate determination results can be obtained.

### 1-3-3. Determination results regarding the risk of cognitive impairment or cognitive decline

The supporting information may include a determination result of risk of cognitive impairment or a determination result of risk of cognitive decline. In the present specification, "risk of cognitive impairment" may mean a likelihood that a person will suffer from cognitive impairment or a likelihood that a person is in a state of suffering from cognitive impairment. In addition, in the present specification, the term "risk of cognitive decline" may mean a likelihood that a person will suffer from cognitive decline or a likelihood that a person is in a state of suffering from cognitive decline.

The determination result of risk of cognitive impairment may be, for example, a determination result indicating the degree of the risk, and more specifically, may include information regarding whether the risk is low or high. For example, the supporting information may include a determination result of risk of humans having MCI or dementia (particularly AD).

The determination result of risk of cognitive decline may be, for example, a determination result indicating the degree of the risk, and more specifically, may include information regarding whether the risk is low or high.

### 1-3-4. Generation of determination results

The determination result of cognitive impairment, cognitive decline, or risk thereof described above may be generated based on the first index value and the second index value. Specific examples of generating the determination result will be described below.

For example, a score for determination may be used to generate the determination result, and the score may be calculated using the first index value and the second index value. The score for determination may be calculated using, for example, a combination of "the first index value" and "a value obtained by performing a predetermined processing on the second index value", or a combination of "a value obtained by performing a predetermined processing on the first index value" and "a value obtained by performing a predetermined processing on the second index value", or a combination of "a value obtained by performing a predetermined processing on the first index value" and "the second index value". The predetermined processing may be weighting processing, such as processing of multiplying or adding a predetermined coefficient. In the calculation, the sum, difference, product, or quotient of the two values forming the combination may be calculated, and the calculated sum, difference, product, or quotient may be used as the score for determination. Particularly preferably, the score for determination may be the sum of the two values.

The calculating processing may be the same calculating processing regardless of whether a person to be judged is male or female, or different calculating processing may be performed depending on the sex.

For example, the predetermined processing may be weighting processing according to the first index value.

More specifically, when the first index value is within a numerical range indicating not having cognitive impairment or cognitive decline, the predetermined processing may be processing with which the effect of "the value obtained by performing a predetermined processing on the first index value" on the score for determination is larger and the effect of "the value obtained by performing a prescribed processing on the second index value" on the score for determination is smaller.

Further, when the first index value is within a numerical range indicating having cognitive impairment or cognitive decline, the predetermined processing may be processing with which the effect of "the value obtained by performing a prescribed processing on the first index value" on the score determination is smaller and the effect of "the value obtained by performing a prescribed processing on the second index value" on the score for determination is larger.

Alternatively, the predetermined processing may be weighting processing according to the second index value.

When the second index value is within a numerical range indicating not more progressing of cognitive impairment or cognitive decline, the predetermined processing may be processing with which the effect of "the value obtained by performing a predetermined processing on the first index value" on the score for determination is larger and the effect of "the value obtained by performing a prescribed processing on the second index value" on the score for determination is smaller.

Further, when the second index value is outside a numerical range indicating more progressing of cognitive impairment or cognitive decline, the predetermined processing may be processing with which the effect of "the value obtained by performing a prescribed processing on the first index value" on the score for determination is smaller and the effect of "the value obtained by performing a prescribed processing on the second index value" on the score for determination is larger.

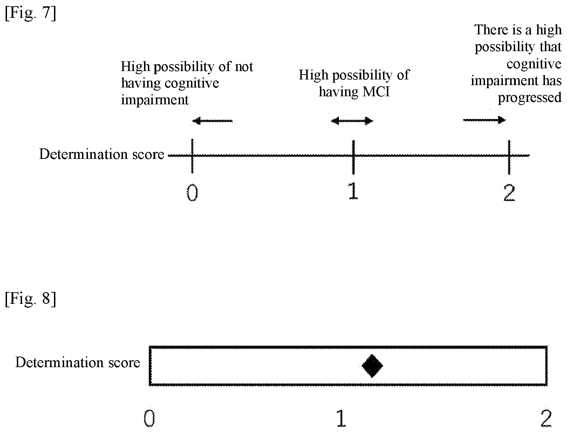

For example, as shown in Fig. 7, the score for determination may be set to have any value in the range of 0 to 2. In this figure, the closer the score for determination is to 2, the more advanced the cognitive impairment in the human is (e.g., having dementia (especially AD), or a possibility of having dementia (especially AD) is high), while the closer the score for determination is to 0, the more the human does not have cognitive impairment (e.g., having neither MCI nor dementia, or a possibility of having neither MCI nor dementia is high). Moreover, when the score for determination is around 1, it indicates that the human has MCI, or a possibility of having MCI is high. The score for determination may be used as an index value indicating these.

Determination of cognitive impairment, cognitive decline, or risk thereof may be performed, based on the score for determination described above. In order to make the determination, for example, the value range of the score for determination may be divided into a plurality of numerical ranges in advance, and a determination result may be associated with each of the plurality of numerical ranges. The number of the multiple numerical ranges may be, for example, 2 to 10, preferably 3 to 8, more preferably 3 to 7.

For example, when the value range of the score for determination is divided into three numerical ranges, one numerical range (for example, a low numerical range) among the three numerical ranges may be associated with a determination result that the human is healthy (or the human have no cognitive impairment, or a risk of having cognitive impairment is low). Another numerical range (for example, an intermediate numerical range) among the three numerical ranges may be associated with a determination result that the human has MCI (or the human has a high risk of having MCI). In addition, yet another numerical range (for example, a high numerical range) among the three numerical ranges may be associated with a determination result that the human has dementia (e.g., AD) (or the human has a high risk of having dementia).

For example, when the value range of the score for determination is divided into four numerical ranges, one numerical range (for example, the lowest numerical range) among the four numerical ranges may be associated with a determination result that the human is healthy (or the human does not have cognitive impairment, or a risk of having cognitive impairment is low). Another numerical range (e.g., the second lowest numerical range) among the four numerical ranges may be associated with a determination result that the human has cognitive decline (or the human has a high risk of having cognitive decline). Another numerical range (e.g., the third lowest numerical range) among the four numerical ranges may be associated with a determination result that the human has MCI (or the human has a high risk of having MCI). Further, still another numerical range (e.g., the highest numerical range) among the four numerical ranges may be associated with a determination result that the human has dementia (e.g., AD) (or the human has a high risk of having dementia).

Alternatively, when the value range of the score for determination is divided into four numerical ranges, one numerical range (for example, the lowest numerical range) among the four numerical ranges may be associated with a determination result that the human is healthy (or the human does not have cognitive impairment, or a risk of having cognitive impairment is low). Another numerical range (e.g., the second lowest numerical range) among the four numerical ranges may be associated with a determination result that the human exhibits characteristic symptoms of early cognitive impairment (e.g., characteristic plasma symptoms). Another numerical range (e.g., the third lowest numerical range) among the four numerical ranges may be associated with a determination result that the human exhibits characteristic symptoms of cognitive impairment (e.g., characteristic plasma symptoms). Further, still another numerical range (e.g., the highest numerical range) among the four numerical ranges may be associated with a determination result that the human exhibits characteristic symptoms of advanced cognitive impairment (e.g., characteristic plasma symptoms).

Alternatively, when the value range of the score for determination is divided into four numerical ranges, one numerical range (for example, the lowest numerical range) among the four numerical ranges may be associated with a determination result that the human has no risk or near no risk of having cognitive impairment (e.g., MCI or AD). Another numerical range (e.g., the second lowest numerical range) among the four numerical ranges may be associated with a determination result that the human has a low risk of having cognitive impairment (e.g., MCI or AD). Another numerical range (e.g., the third lowest numerical range) among the four numerical ranges may be associated with a determination result that the human has an intermediate risk of having cognitive impairment (e.g., MCI or AD). Further, still another numerical range (e.g., the highest numerical range) among the four numerical ranges may be associated with a determination result that the human has a high risk of having cognitive impairment (e.g., MCI or AD).

Generating determination supporting information in which the determination result is associated with the human determination score is useful for supporting determination of cognitive impairment, cognitive decline, or risk thereof.

Further, as a part of the determination supporting information, data describing the correspondence relationship between a plurality of value ranges of the determination score and the determination result in each value range may be included. The data makes the determination result easier to understand. For example, the person or health care professional can know that a higher determination score is associated with a higher risk of cognitive impairment.

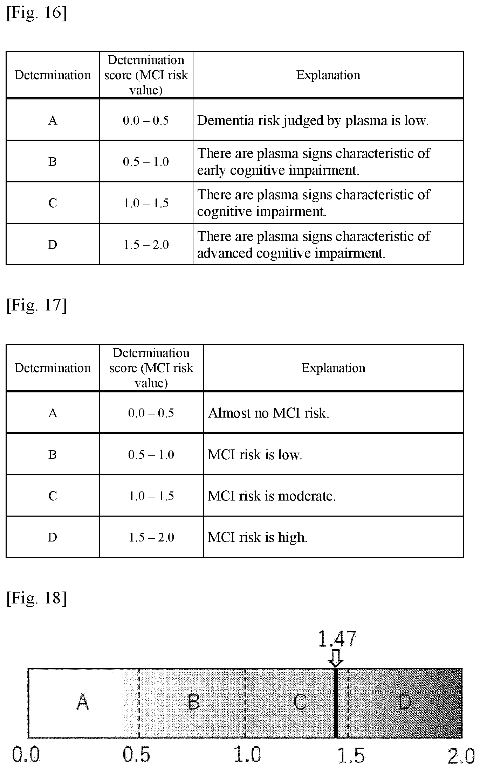

Examples of data describing the correspondence are shown in Figs. 16 and 17. A table containing a plurality of value ranges of a score for determination and descriptions of determination results associated with each value range as shown in Fig. 16 or 17 may be included in the determination supporting information as data describing the corresponding relationship.

The numerical range associated with the determination result that the human has MCI (or the human has a high risk of having MCI) may be further divided into multiple numerical ranges. Each of the plurality of numerical ranges may be associated with a determination result indicating that the patient is in the early stage of MCI, in the middle stage of MCI, or in the late stage of MCI.

In order to make determination based on the score for determination, the information processing device 200 may have a data table including the plurality of numerical ranges in the value range of the score for determination and determination results associated with each of the plurality of numerical ranges. The data table may be stored in the storage unit 202 of the information processing device 200, for example. The information processing device 200 can refer to the data table, select the determination result corresponding to the determination score, and select it as the determination result of the human (or the biological sample).

### 1-3-5. Generation of plot data

### 1-3-5-1. Plotting on one axis

The information processing device 200 may generate plot data indicating the position of the calculated score for determination in the range of the score for determination. For example, as shown in Fig. 8, data indicating one axis indicating the value range and the position of the score for determination on the axis may be generated. Assume that the scores for determination shown in Fig. 8 has the meanings described above with reference to Fig. 7. In this case, the score for determination shown in Fig. 8 (indicated by the black diamond) is slightly higher than 1, but significantly lower than 2. Therefore, according to the plot data shown in Fig. 8, for example, it can be understood that a human having the score for determination has a high possibility of having MCI, or although it has not yet reached the stage of dementia, there is a high possibility that it is transitioning from MCI to the stage of dementia.

Another plot data example is shown in Fig. 18. In the figure, the axis of score for determination in the value range of 0.0 to 2.0 extends in the horizontal direction. The axis is divided into four value range, A, B, C, and D. Among these, the position of the calculated score for determination of 1.47 is indicated by an arrow and a black line. In addition, a color gradation is applied from 0.0 to 2.0 so that it becomes easier to understand that, for example, the risk of cognitive impairment increases as the score for determination increases. Such plot data may be generated in the present invention.

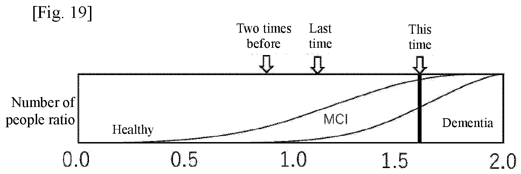

Still another example of plot data is shown in Fig. 19. In the figure, the axis of score for determination in the value range of 0.0 to 2.0 extends in the horizontal direction. In addition, in the figure, the vertical axis indicates the "ratio of the number of people". The number of people ratio indicates, for example, the ratio of people diagnosed healthy or MCI (or dementia) in a human population over a predetermined age. Such number of people ratio may be presented and is useful in understanding the degree of progression of cognitive impairment in humans. Also, in the same figure, the score for determination of "two times before", "last time", and "this time" are indicated by arrows. By including such previous determination results in the plot data, it is possible to know the degree of progression of cognitive impairment.

The plot data may be used as supporting information. Since human cognitive changes gradually, the plot data is useful for understanding the state or transition of human cognitive function.

Alternatively, the plot data may be generated, and the determination may be made based on the positions of the plotted score for determination.

### 1-3-5-2. Plotting into two-dimensional matrix

The first index value and the second index value may be plotted into a two-dimensional matrix to make the determination.

For example, a two-dimensional matrix is prepared in which the X-axis is of the first index value and the Y-axis is of the second index value. Plotting is performed in the two-dimensional matrix using the calculated first index value and second index value. The determination may be made based on the plotted position.

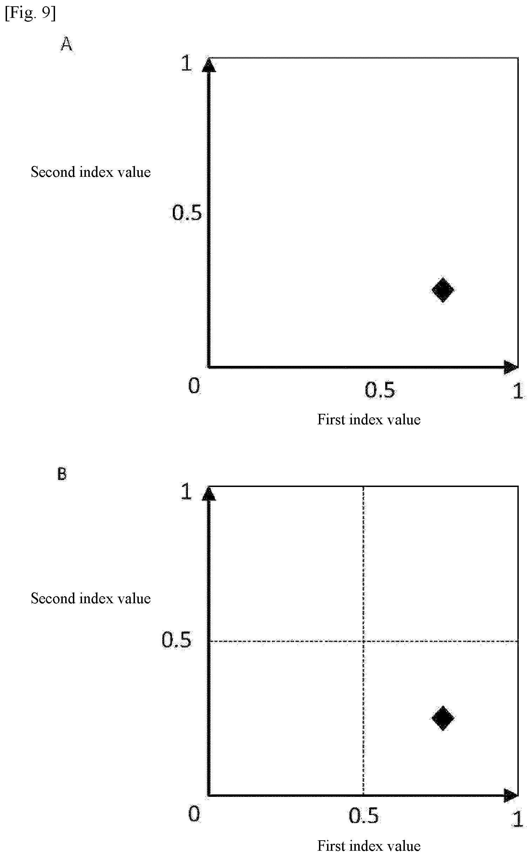

For example, as shown in Fig. 9A, the closer the plotted position is to the upper right in the two-dimensional matrix, the more advanced cognitive impairment is in the human; and the closer to the lower left, the human has no cognitive impairment or the less advanced cognitive impairment is in the human.

For example, it is assumed that the first index value shown in Fig. 9A is the index value as described with reference to Fig. 5 and the second index value is the index value as described with reference to Fig. 6B. In this case, in the positions where these values are plotted (black diamond positions), the first index value that takes a numerical range from 0 to 1 exceeds 0.5 but is less than 1, and the second index value that takes a numerical range from 0 to 1 is less than or equal to 0.5, but greater than 0. Therefore, it can be understood that the biological sample or human from which these first index value and second index value were obtained has cognitive impairment (or has a high possibility of having cognitive impairment) and is in the stage of MCI (has a high possibility of being in the stage of MCI).

Alternatively, the two-dimensional matrix may be divided into four quadrants in advance, as shown in Fig. 9B. Then, when the plotted position exists in the upper right quadrant in the two-dimensional matrix, it can be determined that the human suffers from (or has a high risk of suffering from) dementia (e.g., AD).

Also, if the plotted position is in the lower right quadrant of the two-dimensional matrix, it can be determined that the human has (or is at a high risk of having) MCI.

Further, the human can be judged to be healthy if the plotted position is in the lower left quadrant of the two-dimensional matrix.

In addition, when the plotted position exists in the upper left quadrant, it may not be judged, or may be judged as unjudgeable, for example.

The two-dimensional matrix plot data may be used as supporting information. Since human cognitive function changes gradually, the plot data is useful for understanding the state or transition of human cognitive function.

In the supporting information generating step, as described above, the determination result of cognitive impairment, cognitive decline, or risk of any of them is generated. Therefore, the supporting information generating step may be called a determination step.

### 1-3-6. Determination on the amount of each biomarker

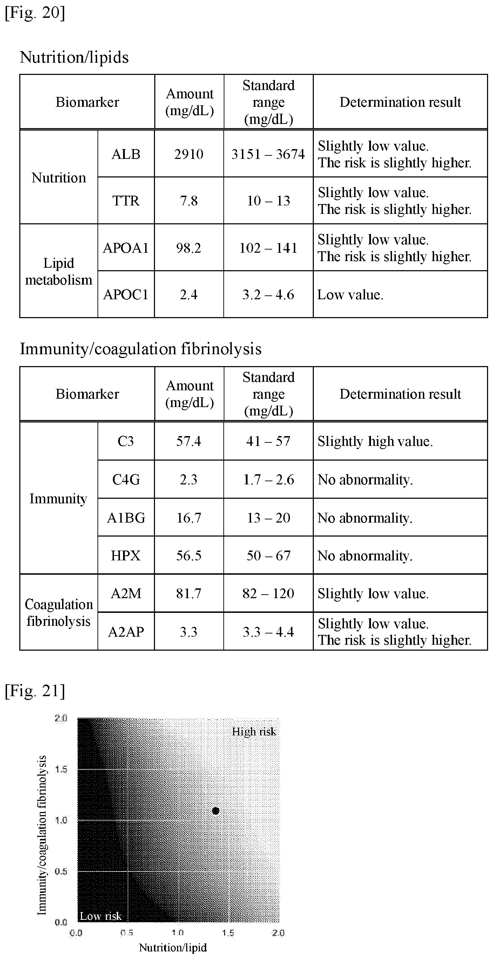

The supporting information may comprise data regarding the amount of each biomarker. The supporting information may further include a determination result based on respective amount of each biomarker. An example of tabular data containing these data is described below with reference to Fig. 20.

The figure shows tabular data containing amount data for each biomarker.

Among the biomarkers shown in the figure, ALB (albumin, above (4)) and TTR (transthyretin, above (10)) are shown as biomarkers indicating nutritional status. These biomarkers are also useful as indicators of nutritional status.

In addition, APOA1 (apolipoprotein A1, above (5)) and APOC1 (apolipoprotein C1, above (6)) are indicated as biomarkers that indicate the state of lipid metabolism. These biomarkers are also useful as biomarkers that indicate the state of lipid metabolism.

In addition, C3 (complement component 3, above (7)), C4G (complement component 4 gamma chain, above (8)), A1BG (alpha-1-B-glycoprotein, above (1)), and HPX (hemopexin, above (9)) have been shown as biomarkers that indicates immune status. These biomarkers are also useful as indicators of immune status.

In addition, A2M (alpha-2-macroglobulin, above (3)) and A2AP (alpha-2-antiplasmin, above (2)) are indicated as biomarkers indicating the state of coagulation and fibrinolysis. These biomarkers are also useful as indicators of the state of coagulation and fibrinolysis.

Thus, the supporting information may include data regarding the amount of each biomarker. This makes it possible to grasp the nutritional status, lipid metabolism status, immune status, and coagulation and fibrinolysis status.

Further, as shown in the figure, in addition to the amount data of each biomarker, a standard numerical range for each biomarker (indicated as "standard range" in the figure) may be included in the table data. Furthermore, as shown in the figure, the table data may include determination results based on amount data of each biomarker. These data allow a more accurate understanding of the subject's condition.

In addition, in the present invention, an index value based on the biomarker amount indicating nutritional status and lipid metabolism status and an index value based on the biomarker amount indicating immune status and coagulation and fibrinolysis status may be calculated. These two index values may be calculated using a predetermined discriminant, like the first index value or the second index value described above. These two index values may be plotted as shown in Fig. 21, for example.

### 1-3-7. Other determination result

The supporting information may include a determination result other than the determination results based on biomarkers. Examples of the determination result include, but are not limited to, test results regarding the subject's genotype.

### 1-4. Output step

In the output step S4, the supporting information generated in the supporting information generating step S3 is output. For example, the information processing device that has executed the supporting information generating step S3 may output the supporting information.

### 1-5. Information processing device that executes index value calculating step, supporting information generating step, and output step

The information processing device that executes the index value calculating step, the supporting information generating step, and the output step may be, for example, an information processing device different from the information processing device included in the biomarker quantification system.

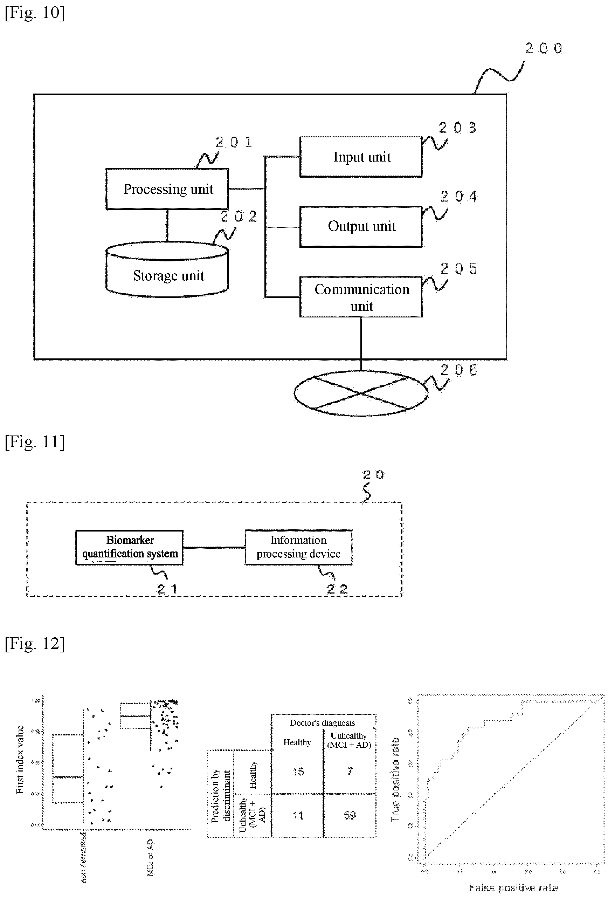

A specific configuration example of the information processing device that executes these steps will be described with reference to Fig. 10. The information processing device 200 shown in Fig. 10 is equipped with a processing unit 201, a storage unit 202, an input unit 203, an output unit 204, and a communication unit 205. For example, a general-purpose computer or server may be used as the information processing device 200.

The processing unit 201 may be configured to execute, for example, an index value calculating step S2 and a supporting information generating step S3 (optionally, an output step S4 in addition to these two steps), and more specifically, it has circuitry configured to perform these steps. Since the above description of the processing unit 101 applies to the more specific configuration of the processing unit 201 (for example, CPU and RAM), the description of the present configuration is omitted.

The processing unit 201 calculates the first index value and the second index value, for example, based on the amount of the two or more biomarkers obtained from the biomarker quantification system (especially the information processing device 100) described above. The calculating processing may be as described in 1-2 above.

The processing unit 201 generates the supporting information, for example, based on the first index value and the second index value. The generating treatment may be as described in 1-3. above.

The processing unit 201 may further be configured to acquire data from the storage unit 202 or record data in the storage unit 202. The storage unit 202 stores various data. The storage unit 202 may be configured to be able to store, for example, the two or more biomarker amount data acquired from the biomarker quantification system, the first index value and the second index value calculated in the index value calculating step S2, and the supporting information generated in the supporting information generating step S3. Further, the storage unit 202 may store a discriminant used to calculate the first index value and the second index value. Further, the storage unit 202 may store the data table used in generating the supporting information and/or information relating to association between the discrimination score and the determination result.

The storage unit 202 can store an operating system (for example, WINDOWS (registered trademark), UNIX (registered trademark), LINUX (registered trademark), or the like), a program for causing the information processing device to execute an index value calculating step S2 and a supporting information generating step S3 (optionally an output step S4), and various other programs. Note that these programs may be recorded in a recording medium, not limited to the storage unit 202.

The input unit 203 can include an interface configured to accept input of various data. The input unit 203 can include, for example, a mouse, a keyboard, a touch panel, and the like as devices that receive such operations.

The output unit 204 can include an interface configured to output various data. For example, the output unit 204 can output the first index and second index values calculated in the index value calculating step S2. Further, the output unit 204 can output the supporting information generated in the supporting information generating step S3. The output unit 204 may include, for example, a display device and/or a printing device as a device for performing the output, and the method of output may be display on a display device or printing by a printing device.

The communication unit 205 can be configured to connect the information processing device 200 to the network by wire or wirelessly. The communication unit 205 allows the information processing device 200 to acquire various data via the network. The acquired data can be stored in the storage unit 202, for example. The configuration of the communication unit 205 may be appropriately selected by those skilled in the art.

The information processing device 200 may include, for example, a drive (not shown) and the like. The drive can read data (for example, the various data mentioned above) or programs recorded on a recording medium and output them to RAM. The recording medium is, for example, a microSD memory card, an SD memory card, or a flash memory, but is not limited to these.

Note that the index value calculating step, the supporting information generating step, and the output step may be performed by one information processing device, or may be performed by two or more information processing devices. For example, the index value calculating step may be performed by one information processing device, and the supporting information generating step and the output step may be performed by another information processing device.

Also, for example, the index value calculating step and/or the supporting information generating step may be executed by an information processing device included in the biomarker quantification system. The output step may be performed by an information processing device included in the biomarker quantification system.

The present technology also provides an information processing device that executes the index value calculating step and the supporting information generating step. The information processing device is as described above.

### 2. Determination supporting information generating system

The present technology also provides a determination supporting information generating system. The system may generate information that supports determination of cognitive impairment, cognitive decline, or risk of any of them. Fig. 11 shows a block diagram of the determination supporting information generating system. As shown in Fig. 11, the determination supporting information generating system 20 includes a biomarker quantification system 21 and an information processing device 22. The determination supporting information generating system may be configured such that the biological sample is subjected to proteolytic degradation treatment and then to measurement by the biomarker quantification system.

The biomarker quantification system 21 may be a system as described in 1-1-2-4. above, and the description also applies to the biomarker quantification system included in the determination supporting information generating system. For example, the biomarker quantification system can comprise a liquid chromatography mass spectrometer.

The information processing device 22 may be a device as described in 1-5. above, and the description also applies to the information processing device included in the determination supporting information generating system.

Note that the present technology also provide an information processing device that executes the index value calculating step and the supporting information generating step described in 1. above.

### 3. Combination of biomarkers

The present technology also provides combinations of two or more of the biomarkers (1) to (10) above. The combination may be used for determining cognitive impairment, cognitive decline, or risk of any of them.

The present technology also provides a combination of two or more biomarkers among the biomarkers of (1), (4), (8), and (9) above. That is, the combination may contain two, three, or all four biomarkers among the biomarkers (1), (4), (8), and (9). Combinations of these biomarkers may be used for determining cognitive impairment, cognitive decline, or risk of any of them, particularly for determining the presence or absence of cognitive impairment or cognitive decline.

Particularly preferably, the combination further comprises one, two, three, or four biomarkers among the biomarkers of (2), (3), (6) and (7) in addition to the biomarkers (1), (4), (8) and (9). Combinations of these biomarkers are particularly suitable for determining the presence or absence of cognitive impairment or cognitive decline.

The combination of biomarkers may also be used for determining cognitive impairment, cognitive decline, or the risk of any of these, in particular for determining the degree of progression of cognitive impairment or cognitive decline. These biomarker combinations are particularly suitable for determining the degree of progression of cognitive impairment or cognitive decline.

### 4. Kit

The present technology also provides a biological sample collection kit for use in performing the method of generating determination supporting information according to the present technology. The biological sample collection kit may be configured, for example, to be able to collect human blood. For example, the biological sample collection kit includes a needle for blood collection and a container that holds blood collected through the needle. The container may, for example, contain an absorbent material (such as paper, in particular filter paper, or a sponge-like structure, etc.) that is soaked with the blood.

The biological sample collection kit may be configured to allow delivery of a biological sample to, for example, the biomarker quantification system 10 described above. For example, the biological sample collection kit (particularly the container of the kit) can be configured to be received in the receiving unit included in the biomarker quantification system 10.

### 5. Discriminant derivation method

The present technology also provides a method of deciding a discriminant for determining cognitive impairment, cognitive decline, or risk of any of them. The deciding method can include a step of acquiring data regarding amounts of biomarkers constituting the combination contained in a biological sample in each of a plurality of humans, and a discriminant derivation step of deriving a discriminant by performing regression analysis using the presence or absence of cognitive impairment or cognitive decline and/or the stage of cognitive impairment or cognitive decline for each of the plurality of humans, and the amounts measured for respective human biological samples. The discriminant derivation method may include a detection step of using the derived discriminant to detect cognitive impairment, cognitive decline, or risk of any of them in the subject.

The regression analysis performed in the discriminant derivation step is, for example, logistic regression analysis, but is not limited thereto, and may be other analysis methods described in 1. above. In the discriminant, the objective variable may be, for example, the presence or absence of cognitive impairment or the stage of cognitive impairment. In the discriminant, the explanatory variable may be, for example, the amounts of biomarkers that make up the combination according to the present technology.

### 6. Determination supporting information generating method using multiple types of biomarkers for determination

The present technology also provides a method for generating determination supporting information, comprising executing a supporting information generating step for generating information that supports determination of cognitive impairment, cognitive decline, or risk of any of them in the human, based on amounts of two or more biomarkers among the following biomarkers (A) to (D) contained in a human-derived biological sample:
(A) at least one biomarker for determining nutritional status;
(B) at least one biomarker for determining lipid composition;
(C) at least one biomarker for determining immune function, and
(D) at least one biomarker for determining the coagulation and fibrinolysis system.

The combination of two or more biomarkers among these four biomarkers is useful for determining cognitive impairment, cognitive decline, or risk of any of them in humans. Each of these biomarkers is also useful for nutritional status determination, lipid composition determination, immune function determination, and coagulation and fibrinolysis system determination. Therefore, by these biomarkers, determination on cognitive impairment or cognitive decline in humans can be performed and determination on two or more of nutritional status, lipid composition, immune function, and the coagulation and fibrinolytic system can be performed. That is, according to the present technology, in addition to the generation of information supporting determination of cognitive impairment or cognitive decline in humans, simultaneously, determination supporting information on two or more among nutritional status, lipid composition, immune function, and coagulation and fibrinolysis system can be generated. These information can also encourage humans to improve lifestyle to maintain or improve cognitive function.

The biomarker (A) may comprise at least one of the following biomarkers (a1) and (a2):
(a1) albumin or its partial peptide, and
(a2) transthyretin or its partial peptide.

The biomarkers (a1) and (a2) are the same as the biomarkers (4) and (10) described in 1. above, respectively, and the description also applies to this supporting information generating method.

The (B) biomarker may comprise at least one of the following biomarkers (b1) and (b2):
(b1) apolipoprotein A1 or its partial peptide, and
(b2) apolipoprotein C1 or its partial peptide.

The biomarkers (b1) and (b2) are the same as the biomarkers (5) and (6) described in 1. above, respectively, and the description also applies to this supporting information generating method.

The (C) biomarker may comprise at least one of the following biomarkers (c1) to (c4):
(c1) complement component 3 or its partial peptide,
(c2) complement component 4 gamma chain or its partial peptide,
(c3) alpha-1-B-glycoprotein or its partial peptide, and
(c4) hemopexin or its partial peptide.

The biomarkers (c1), (c2), (c3), and (c4) are the same as the biomarkers (7), (8), (1), and (9) described in 1. above, respectively, and the description also applies to this supporting information generating method.

The (D) biomarker may comprise at least one of the following biomarkers (d1) and (d2):
(d1) alpha-2-macroglobulin or its partial peptide, and
(d2) alpha-2-antiplasmin or its partial peptide.

The biomarkers (d1) and (d2) are the same as the biomarkers (3) and (2) described in 1. above, respectively, and the description also applies to this supporting information generating method.

The determination supporting information generating method may further include a data acquisition step of acquiring data regarding amounts of each biomarker. The data acquisition step may be performed in the same manner as the data acquisition step described in 1-1. above.

Further, the determination supporting information generating method may include an output step of outputting the supporting information generated in the supporting information generating step. The output step may be performed in the same manner as the output step described in 1-5. above.

### EXAMPLES

Hereinafter, the present technology will be described in further detail based on examples. The examples described below show examples of typical examples of the present technology, and the scope of the present technology is not limited only to these examples.

### 7. Example 1

### 7-1. Creation of discriminants used to calculate first index value and second index value

Ninety-two cases were prepared that were diagnosed by a physician regarding cognitive function. Of the 92 cases, 26 were healthy and 66 had cognitive impairment. Cases with cognitive impairment were also diagnosed with either MCI or dementia of the Alzheimer's type.

Biological samples (plasma) were obtained from each of the 92 cases. Then, amounts of the following biomarkers (1) to (10) contained in each plasma were quantified.
(1) alpha-1-B-glycoprotein;
(2) alpha-2-antiplasmin;
(3) alpha-2-macroglobulin;
(4) albumin;
(5) apolipoprotein A1;
(6) apolipoprotein C1;
(7) complement component 3;
(8) complement component 4 gamma chain;
(9) hemopexin; and
(10) transthyretin.

Pretreatment of plasma conducted for quantification of the amounts of biomarkers and measurement conditions for the biomarker quantification after the pretreatment are described below.

### (Pretreatment)

1. 3 µl of plasma was mixed with 22.5 µl of a heat denaturation solution (company name: Nacalai Tesque, product name: CHAPS) and heat denatured at 99.9 °C for 5 minutes in a thermal cycler.
2. 8.5 µl of the heat-denatured plasma-containing solution was mixed with 43.5 µl of a trypsin solution (company name: Promega, product name: Sequencing Grade Modified Trypsin, trypsin concentration: about 20 ng/µl). The plasma-containing solution after addition of the trypsin solution was incubated at 37°C for 16 hours.
3. After the incubation, 140 µl of a formic acid solution (company name: FUJIFILM Wako chemicals, product name: formic acid, formic acid concentration: 1%) was added to stop the enzymatic reaction.
4. After stopping the enzymatic reaction, a stable isotope-labeled internal standard peptide used for quantification by a liquid chromatography mass spectrometer, which will be described later, was added to the plasma-containing solution to prepare a sample for analysis.

### (Quantification of biomarkers by liquid chromatography mass spectrometer)

Biomarker quantification was performed by a liquid chromatography mass spectrometer, using 2 µl of the sample for analysis obtained by the pretreatment. The liquid chromatography mass spectrometer was an LCMS-8060 system manufactured by Shimadzu Corporation. A reverse-phase column (AERIS PEPTIDE XB-C18, manufactured by Phenomenex) was attached to the system, separation was performed by reverse-phase chromatography (0.1% formic acid, 2% to 90% acetonitrile), and mass spectrometry was performed.

The mass spectrometry was performed according to the MRM (multiple reaction monitoring) method. The measurement conditions for each biomarker in the MRM method are shown in Table 2 below.

### [Table 2]

**Table 2: MRM conditions**

| | | | | | | | | | | |
|---|---|---|---|---|---|---|---|---|---|---|
| Biomarker number | Biomarker abbreviation | Holding time (min) | Presence or absence of signs | Amino acid sequence of quantitative fragment | For quantification (m/z) | | For confirmation 1 (m/z) | | For confirmation 2 (m/z) | |
| | | | | | Precursor | Product | Precursor | Product | Precursor | Product |
| (1) | AlBG | 7.3 | unlabeled | LETPDFQLFK | 619.35 | 243.15 | 619.35 | 894.45 | 619.35 | 215.15 |
| | | | labeled | LETPDFQLF[K] | 623.35 | 243.15 | 623.35 | 902.50 | 623.35 | 215.15 |
| (2) | A2AP | 3.9 | unlabeled | LGNQEPGGQTALK | 656.85 | 771.45 | 656.85 | 542.25 | 656.85 | 900.50 |
| | | | labeled | LGNQEPGGQTAL[K] | 660.85 | 779.45 | 660.85 | 542.25 | 660.85 | 908.50 |
| (3) | A2M | 8.1 | unlabeled | LLIYAVLPTGDVIGDSAK | 615.70 | 586.80 | 615.70 | 530.25 | 615.70 | 673.45 |
| | | | labeled | LLIYAVLPTGDVIGDSA[K] | 618.35 | 590.80 | 618.35 | 534.30 | 618.35 | 673.45 |
| (4) | ALB | 5.5 | unlabeled | LVNEVTEFAK | 575.30 | 185.15 | 575.30 | 937.45 | 575.30 | 213.15 |
| | | | labeled | LVNEVTEFA[K] | 579.30 | 185.15 | 579.30 | 945.50 | 579.30 | 213.15 |
| (5) | APOA1 | 3.6 | unlabeled | ATEHLSTLSEK | 405.90 | 572.80 | 405.90 | 522.25 | 405.90 | 363.20 |
| | | | labeled | ATEHLSTLSE[K] | 408.55 | 576.80 | 408.55 | 526.30 | 408.55 | 371.20 |
| (6) | APOC1 | 4.4 | unlabeled | TPDVSSALDK | 516.75 | 466.25 | 516.75 | 834.40 | 516.75 | 620.35 |
| | | | labeled | TPDVSSALD[K] | 520.75 | 470.25 | 520.75 | 842.45 | 520.75 | 628.35 |
| (7) | C3 | 4.6 | unlabeled | TGLQEVEVK | 501.80 | 731.40 | 501.80 | 603.35 | 501.80 | 422.75 |
| | | | labeled | TGLQEVEV[K] | 505.80 | 739.40 | 505.80 | 611.35 | 505.80 | 426.75 |
| (8) | C4G | 5.0 | unlabeled | ITQVLHFTK | 362.90 | 487.30 | 362.90 | 436.75 | 362.90 | 215.15 |
| | | | labeled | ITQVLHFT[K] | 365.55 | 491.30 | 365.55 | 440.75 | 365.55 | 215.15 |
| (9) | HPX | 6.5 | unlabeled | NFPSPVDAAFR | 610.80 | 480.25 | 610.80 | 234.10 | 610.80 | 959.50 |
| | | | labeled | NFPSPVDAAF[R] | 615.80 | 485.25 | 615.80 | 234.10 | 615.80 | 969.50 |
| (10) | TTR | 5.9 | unlabeled | AADDTWEPFASGK | 697.80 | 606.30 | 697.80 | 921.45 | 697.80 | 735.35 |
| | | | labeled | AADDTWEPFASG[K] | 701.80 | 614.35 | 701.80 | 929.45 | 701.80 | 743.40 |

In Table 2 above, the "holding time (min)" column is the holding time of the quantitative fragment of each biomarker in the reversed-phase chromatography. In the "presence or absence of signs" column, "unlabeled" indicates quantitative fragments without labeling (unlabeled peptides), i.e., corresponds to quantitative fragments contained in plasma. In the "presence or absence of signs", "labeled" indicates a quantitative fragment having stable isotope-labeled amino acid residues, i.e., a stable isotope-labeled internal standard peptide. The column "amino acid sequence of quantitative fragment" shows the amino acid sequence (single-letter code) of the quantitative fragment, and the amino acid residues in parentheses are stable isotope-labeled amino acid residues. That is, [K] and [R] at the ends of the amino acid sequence are isotope-labeled sites of the stable isotope peptide for internal standard.

LC-MS signals of quantified fragments of each biomarker were identified using the software Skyline (MacCoss Lab, University of Washington).

The mass spectrometry measured the molarity of biomarker quantitative fragments in the analytical sample. For the measurement, a calibration curve was prepared using a sample group in which an internal standard peptide was added to a dilution series of unlabeled peptide. Using the calibration curve, the measurement was performed by the internal standard method.

For each biomarker, the measured quantitative fragment concentration, the quantitative fragment mass, and the biomarker mass were used to quantify the biomarker amount (molarity) in the sample for analysis.

Quantification of biomarker amounts as described above was performed on biological samples obtained from each of the above 92 cases. The biomarker amounts of the 92 cases were normalized using the mean and standard deviation of the biomarker amounts.

For the above 92 cases, logistic regression analysis was performed with the standardized biomarker amounts of the biomarker group including (1), (4), (8), and (9) among the biomarkers (1) to (10) as an explanatory variable and with information as to whether each case was healthy, MCI, or AD as an objective variable. Through the regression analysis, a discriminant regarding discrimination between healthy and MCI and a discriminant regarding discrimination between healthy and AD were obtained. These discriminants were used to calculate the first index value and the second index value. The first index value is the sum of the probability regarding MCI and the probability regarding AD calculated using the values obtained using these discriminants. The second index value is the probability of having the AD.

### 7-2. Evaluation of created discriminant (discrimination by first index value)

The biomarker amounts of each of the 92 cases was substituted into the discriminant to obtain the first index value of each case. Fig. 12 shows a boxplot plotting the first index value of each case, a confusion matrix regarding the determination result based on the discriminant and the diagnosis result by the doctor, and a receiver operating characteristic curve regarding the determination result based on the discriminant, that is, ROC (receiver operating characteristic curve).

From the results shown in Fig. 12, it can be seen that the first index value is useful for discriminating between humans with healthy cognitive function and humans with cognitive impairment. For example, the accuracy calculated from the confusion matrix was 80%. The AUC (area under the curve) value of the ROC curve was 0.86.

### 7-3. Evaluation of created discriminant (discrimination by second index value)

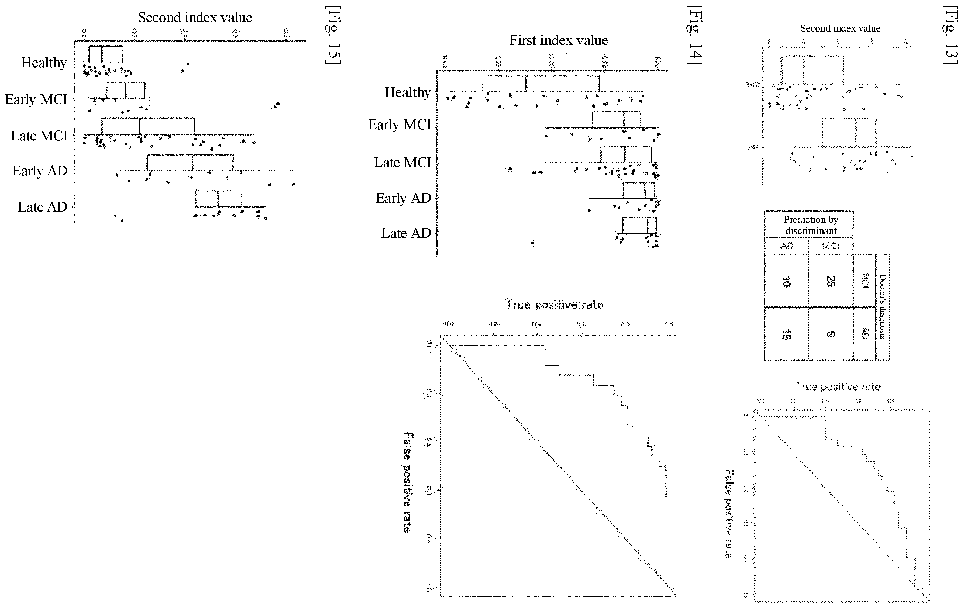

The biomarker amounts of each of the 59 cases was substituted into the discriminant to obtain the second index value of each case. Fig. 13 shows a boxplot plotting the second index value of each case, a confusion matrix relating to the determination result based on the discriminant and the diagnosis result by the doctor, and a ROC curve relating to the determination result based on the discriminant.

From the results shown in Fig. 13, it can be seen that the second index value is useful for distinguishing between MCI and AD. For example, the accuracy calculated from the confusion matrix was 68%. Further, the AUC value of the ROC curve was 0.77.

### 7-4. Usefulness of first index value and second index value

It can be seen that by using the first index value and the second index value according to the present technology, it is possible to accurately determine cognitive impairment or its risk, as described in 7-1. to 7-3. above. Further, it can also been understood that by using the first index value and the second index value, it is possible to determine the degree of progression of human cognitive impairment (for example, whether it is in the stage of MCI or AD).

In addition, the human cognitive function gradually transitions from a healthy stage to a cognitive decline stage and then to a cognitive impairment stage. As described above, it is also possible, for example, by the first index value and the second index value, to determine that the human cognitive function is in a healthy stage, but is close to the cognitive impairment stage, or to determine that the risk of reaching the cognitive impairment stage is high. Therefore, it is also considered that the first index value and the second index value according to the present technology can accurately determine cognitive decline or its risk.

### 8. Example 2

### 8-1. Creation of discriminant used to calculate first index value and second index value

A large number of cases diagnosed by a physician regarding cognitive function were prepared. Each case was diagnosed as healthy, early MCI, late MCI, early AD, or late AD.

A biological sample (plasma) was obtained from each of the cases. Then, amounts of the following biomarkers (1) to (10) contained in each plasma were quantified as described in 7-1. above, to obtain normalized biomarker amounts.

For the above cases, logistic regression analysis was performed with the standardized biomarker amount of the biomarker group including (1), (4), (8), and (9) among the biomarkers (1) to (10) as an explanatory variable and with the information as to whether each case was healthy, MCI, or AD as an objective variable. Through the regression analysis, a discriminant regarding discrimination between healthy and MCI and a discriminant regarding discrimination between healthy and AD were obtained. These discriminants were used to calculate the first index value and the second index value. The first index value is the sum of the probability regarding MCI and the probability regarding AD calculated using the values obtained using these discriminants. A second index value is the probability of having the AD.

### 8-2. Evaluation of created discriminant (discrimination by first index value)

The first index value for each case was obtained by substituting the biomarker amounts of each case into the discriminant. Fig. 14 shows a boxplot plotting the first index value of each case and a ROC curve regarding the judgment result based on the discriminant.

From the results shown in Fig. 14, it can be seen that the first index value is useful for discriminating between humans with healthy cognitive function and humans with cognitive impairment. For example, the AUC value of the ROC curve was 0.865.

### 8-3. Evaluation of created discriminant (discrimination by second index value)

The second index value for each case was obtained by substituting the biomarker amounts of each case into the discriminant. Fig. 15 shows a boxplot plotting the second index value of each case.

From the results shown in Fig. 15, it can be seen that the second index value tends to increase as cognitive impairment progresses. Therefore, it can be seen that the second index value is useful for determining the degree of progression of cognitive impairment. For example, it can be seen that the second index value is useful as an index of which stage of early MCI, late MCI, early AD, and late AD a human is present.

### 8-4. Usefulness of first index value and second index value

It can be seen that by using the first index value and the second index value according to the present technology, it is possible to accurately judge cognitive impairment or its risk, as described in 8-1. to 8-3. above. Further, it can also be seen that it is possible to determine the degree of progression of human cognitive impairment (for example, whether a person is in early MCI stage, late MCI stage, early AD stage, or late AD stage) by using the first index value and the second index value.

### [Description of symbols]

10: Biomarker quantification system
20: Determination supporting information generating system

## Claims

1. A determination supporting information generating method, comprising:
an index value calculating step of calculating, based on amounts of two or more biomarkers contained in a biological sample derived from a human,
(I) a first index value indicating the presence or absence of cognitive impairment or cognitive decline in the human and
(II) a second index value indicating the degree of progression of cognitive impairment or cognitive decline in the human; and
a supporting information generating step of generating information that supports determination of cognitive impairment, cognitive decline, or risk of any of them, based on the first index value and the second index value.

2. The determination supporting information generating method according to claim 1, wherein the amount of the two or more biomarkers used in the index value calculating step includes amounts of two or more biomarkers among the following biomarkers (1) to (10):
(1) alpha-1-B-glycoprotein or its partial peptide;
(2) alpha-2-antiplasmin or its partial peptide;
(3) alpha-2-macroglobulin or its partial peptide;
(4) albumin or its partial peptide;
(5) apolipoprotein A1 or its partial peptide;
(6) apolipoprotein C1 or its partial peptide;
(7) complement component 3 or its partial peptide;
(8) complement component 4 gamma chain or its partial peptide;
(9) hemopexin or its partial peptide; and
(10) transthyretin or its partial peptide.

3. The determination supporting information generating method according to claim 2, wherein the first index value is calculated based on amounts of two or more biomarkers among the biomarkers (1), (2), (3), (4), (6), (7), (8), and (9).

4. The determination supporting information generating method according to claim 2 or 3, wherein the second index value is calculated based on amounts of two or more biomarkers among the biomarkers (1), (2), (3), (4), (6), (7), (8), and (9).

5. The determination supporting information generating method according to any one of claims 1 to 4, wherein the first index value is an index value indicating whether the human has mild cognitive impairment or dementia.

6. The determination supporting information generating method according to any one of claims 1 to 5, wherein the second index value is an index value indicating whether the cognitive impairment of the human is in a stage of mild cognitive impairment or in a stage of dementia.

7. A determination supporting information generating system comprising an information processing device that executes:
an index value calculating step of calculating, based on amounts of two or more biomarkers contained in a biological sample derived from a human,
(I) a first index value indicating the presence or absence of cognitive impairment or cognitive decline in the human and
(II) a second index value indicating the degree of progression of cognitive impairment or cognitive decline in the human; and
a supporting information generating step of generating information that supports determination of cognitive impairment, cognitive decline, or risk of any of them, based on the first index value and the second index value.

8. The system according to claim 7, further comprising a biomarker quantification system that quantifies the amounts of the two or more biomarkers.

9. The system according to claim 8, wherein the biomarker quantitation system comprises a liquid chromatography mass spectrometer.

10. The system according to claim 8 or 9, wherein the system is configured such that the biological sample is subjected to proteolytic degradation treatment and subjected to measurement by the biomarker quantification system.

11. An information processing device that executes:
an index value calculating step of calculating, based on amounts of two or more biomarkers contained in a biological sample derived from a human,
(I) a first index value indicating the presence or absence of cognitive impairment or cognitive decline in the human and
(II) a second index value indicating the degree of progression of cognitive impairment or cognitive decline in the human; and
a supporting information generating step of generating information that supports determination of cognitive impairment, cognitive decline, or risk of any of them, based on the first index value and the second index value.
